# EUROPEAN PATENT SPECIFICATION

(11) **EP 3 512 537 B1**
(45) Date of publication and mention of the grant of the patent: **01.11.2023**
(21) Application number: 16769929.7
(22) Date of filing: 15.09.2016
(51) Int. Cl.: A61K 38/16, A61K 39/02, A61K 39/39, C12N 15/85

(54) **T-CELL IMMUNOTHERAPY**
T-ZELL-IMMUNTHERAPIE
IMMUNOTHÉRAPIE À LYMPHOCYTES T

(43) Date of publication of application: 24.07.2019
(73) Proprietor: Elicera Therapeutics AB, 740 20 Vänge (SE)
(72) Inventor: YU, Di, 754 22 Uppsala (SE); ESSAND, Magnus, 740 20 Vänge (SE); JIN, Chuan, 754 22 Uppsala (SE); RAMACHANDRAN, Mohanraj, 752 73 Uppsala (SE); MA, Jing, 752 71 Uppsala (SE)
(74) Representative: Barker Brettell Sweden AB
(86) International application number: PCT/EP2016/071775
(87) International publication number: WO 2018/050225

(56) References cited:
- EP-A1- 1 767 214
- MARKUS CHMIELEWSKI ET AL: "Of CARs and TRUCKs: chimeric antigen receptor (CAR) T cells engineered with an inducible cytokine to modulate the tumor stroma", IMMUNOLOGICAL REVIEWS, vol. 257, no. 1, 13 January 2014 (2014-01-13), pages 83-90, XP55185690, ISSN: 0105-2896, DOI: 10.1111/imr.12125
- MOHANRAJ RAMACHANDRAN ET AL: "An Infection-enhanced Oncolytic Adenovirus Secreting H. pylori Neutrophil-activating Protein with Therapeutic Effects on Neuroendocrine Tumors", MOLECULAR THERAPY, vol. 21, no. 11, 2 July 2013 (2013-07-02), pages 2008-2018, XP55316355, GB ISSN: 1525-0016, DOI: 10.1038/mt.2013.153
- M. RAMACHANDRAN ET AL: "Vector-Encoded Helicobacter pylori Neutrophil-Activating Protein Promotes Maturation of Dendritic Cells with Th1 Polarization and Improved Migration", THE JOURNAL OF IMMUNOLOGY, vol. 193, no. 5, 21 July 2014 (2014-07-21) , pages 2287-2296, XP55316375, US ISSN: 0022-1767, DOI: 10.4049/jimmunol.1400339
- AMEDEI AMEDEO ET AL: "The neutrophil-activating protein of Helicobacter pylori promotes Th1 immune responses", JOURNAL OF CLINICAL INVESTIGATION, AMERICAN SOCIETY FOR CLINICAL INVESTIGATION, US, vol. 116, no. 4, 1 April 2006 (2006-04-01) , pages 1092-1101, XP002421963, ISSN: 0021-9738, DOI: 10.1172/JCI27177
- GAIA CODOLO ET AL: "HP-NAP inhibits the growth of bladder cancer in mice by activating a cytotoxic Th1 response", CANCER IMMUNOLOGY, IMMUNOTHERAPY, SPRINGER, BERLIN, DE, vol. 61, no. 1, 11 August 2011 (2011-08-11), pages 31-40, XP019995548, ISSN: 1432-0851, DOI: 10.1007/S00262-011-1087-2

## Description

### TECHNICAL FIELD

The present embodiments generally relate to vectors and T-cells, and in particular to such T-cells useful in T-cell immunotherapy and in treatment of cancer.

### BACKGROUND

Immunotherapy is currently facing major breakthroughs in the management of cancer. The success stories of monoclonal antibody therapy with HERCEPTIN^{®} (anti-Her2) for breast cancer and MabThera^{®} (anti-CD20) for B-cell malignancies have paved way for more sophisticated immunotherapies, such as PROVENGE^{®}, a dendritic cell (DC) vaccine for metastatic prostate cancer. Recently, immunotherapy with monoclonal antibodies targeting the immune system rather than tumor cells has proven successful. Monoclonal anti-CTLA4 (cytotoxic T-lymphocyte-associated protein 4) antibodies and anti-PD1 (programmed cell death protein 1) antibodies are effective in releasing the break for T-cells to thereby make them attach to tumor cells. The potential of therapeutic T-cells to traffic to sites of disease, expand and persist following a single injection remain major advantages over monoclonal antibodies. In fact, cancer immunotherapy with adoptive transfer of tumor infiltrating T-cells (TILs) can in many cases lead to cure [1]. Alternatively, if the tumor associated antigen (TAA) is known, bulk T-cells can be isolated from peripheral blood of cancer patients for genetic engineering with molecules recognizing the tumor associated antigen where after they are re-infused to the patient.

T-cells can be engineered to express a T-cell receptor (TCR) or a chimeric antigen receptor (CAR), recognizing a tumor associated antigen. Recent publications from ongoing trials demonstrate that such designed T-cells can induce complete remission in patients with otherwise refractory cancer [2-4].

Successful attempt was observed with a CAR against CD19 in patients with advanced B-cell chronic lymphocytic leukemia (CLL) and B-cell acute lymphocytic leukemia (ALL), with CAR T-cells proliferating extremely well upon adoptive transfer [2, 4, 5].

To a lesser extent clinical success has also been observed for refractory B-cell lymphomas. The lesser success for lymphomas than for leukemia can be attributed to the lumpy structure of lymphomas in lymph nodes and organs as compared to leukemic tumor cells mostly found circulating in the blood of cancer patients [6, 7].

Despite encouraging results, several immunological characteristics of the tumor microenvironment (TME) restrict effective CAR T-cells immunotherapy, especially in the case of solid tumors. The outcome in solid tumors is limited by, among others, the complex structure of tumors and their immunosuppressive microenvironment. Besides malignant cells most solid tumors also consist of several populations of nonmalignant cells: fibroblasts, mesenchymal cells and various immune cells, together with extracellular matrix and inflammatory mediators. In many cases the malignant cells are in minority within a tumor. The endothelial cells in the tumor bed can also act as a barrier by blocking effector T-cells from entering while letting regulatory T-cells pass. Human cancer cells can evolve and escape immune recognition by downregulating TAA and most tumors are heterogenous. Furthermore, in solid tumors there are immunosuppressive cells, cytokines and inhibitory molecules that can interfere with the success of CAR T-cell immunotherapy.

Accordingly, CAR T-cell immunotherapy has, up to know, mainly been effective in blood-borne cancers, such as leukemia. There is therefore a need for an improved T-cell immunotherapy that is effective also for solid tumors.

### SUMMARY

An objective of the embodiments is to provide T-cells useful in T-cell immunotherapy.

This and other objectives are met by embodiments as disclosed herein.

The present invention is defined in the independent claims. Further embodiments of the present invention are defined in the dependent claims.

An aspect of the embodiments relates to a vector comprising a nucleic acid sequence encoding a chimeric antigen receptor (CAR) and/or a nucleic acid sequence encoding a T-cell receptor (TCR). The vector also comprises a nucleic acid sequence encoding a *Helicobacter pylori* neutrophil activating protein (HP-NAP) and/or a nucleic acid sequence encoding an immunologically equivalent fragment of HP-NAP. The immunologically equivalent fragment of the HP-NAP is a fragment including active polypeptide domains of at least 20-40 amino acid residues of the HP-NAP, preferably selected from the group consisting of SEQ ID NO: 9 to 12.

Another aspect of the embodiments relates to a T-cell comprising a CAR, a TCR, a nucleic acid sequence encoding CAR and/or a nucleic acid sequence encoding TCR. The T-cell also comprises a HP-NAP, an immunologically equivalent fragment of HP-NAP, a nucleic acid sequence encoding HP-NAP and/or a nucleic acid sequence encoding an immunologically equivalent fragment of HP-NAP. The immunologically equivalent fragment of the HP-NAP is a fragment including active polypeptide domains of at least 20-40 amino acid residues of the HP-NAP, preferably selected from the group consisting of SEQ ID NO: 9 to 12.

A further aspect of the embodiments relates to a pharmaceutical composition comprising a T-cell according to above and a dendritic cell, preferably an immature dendritic cell.

Additional aspects of the embodiments relates to a T-cell according to above or a pharmaceutical composition according to above for use as a medicament, for use in T-cell immunotherapy or for use in treating, reducing and/or preventing cancer.

A further aspect of the embodiments relates to a method of inducing maturation of immature dendritic cells. The method comprising contacting, preferably *in vitro,* the immature dendritic cells with a T-cell according to above.

The T-cells of the present embodiments have improved cytotoxicity and stimulate chemokine and cytokine secretion and promote dendritic cell maturation. The T-cells further induced Th1 polarization and recruited and active innate immune cells.

### BRIEF DESCRIPTION OF THE DRAWINGS

The embodiments, together with further objects and advantages thereof, may best be understood by making reference to the following description taken together with the accompanying drawings, in which:

Otherwise specifically indicated in each section, all experiments were performed six times with CAR T-cells produced from six different healthy blood donors. Error bars represent SEM and statistical significance is depicted by symbols (ns, no statistical significance, *: P<0.05, **: P<0.01, ***: P<0.001).
Figure 1. Schematic illustration of the lentiviral construct used to transduce T-cells.
Figure 2. Schematic illustration of NFAT-IL-2 promoter activation and transgene expression upon target cell recognition.
Figure 3. T-cells were transduced with LV(nLuc-CD19CAR) and expanded for two weeks. Transduced T-cells alone or transduced T-cells stimulated with Daudi target cells cells at a ratio of 5:1 was assessed by nano-luciferases release after 24 hours.
Figure 4. T-cells were transduced with LV(nLuc-CD19CAR) and expanded for two weeks. T-cells were intravenously injected together with CD19+ Daudi or CD19 BC-3 at a ratio 2:1 into NOD-SCID mice (3 mice/group) on day 0. Nano-Luc expression was monitored on days 3 and 5.
Figure 5. T-cells were transduced with LV(CD19CAR) or LV(NAP-CD19CAR) and expanded for two weeks. Flow cytometry was used to analyze NAP expression from NAP-CD19CAR T-cells co-cultured with Daudi cells at a ratio of 5:1. Data are shown as double NAP⁺CD19CAR⁺ positive T-cells out of total CD3⁺ cells.
Figure 6. T-cells were transduced with LV(CD19CAR) or LV(NAP-CD19CAR) and expanded for two weeks. Representative histograms demonstrating NAP expression from CD19CAR⁺ T-cells.
Figure 7. Schematic illustration of the experiments as presented in Figures 8-11.
Figure 8. T-cells were transduced with LV(Mock), LV(CD19CAR), LV(NAP-CD19CAR) respectively and expanded for two weeks. The cytotoxic ability of transduced and expanded T-cells was evaluated by co-culturing them with firefly luciferase-tagged Daudi target cells at a ratio of 5:1 or by co-culture with autologous imDCs and firefly luciferase-tagged Daudi cells at a ratio of 5:1:1 after 4 days. Relative viability of target cells were measured by luminescence and related to signals from target cells alone.
Figure 9. T-cells were transduced with LV(Mock), LV(CD19CAR), LV(NAP-CD19CAR) respectively and expanded for two weeks. CD19CAR T-cells were co-cultured with Daudi cells at a ratio of 5:1 or co-cultured with imDCs and Daudi cells at a ratio of 5:1:1 for 20-24 hours. ELISA was used to analyze IFN-γ production from supernatant.
Figure 10. T-cells were transduced with LV(Mock), LV(CD19CAR), LV(NAP-CD19CAR) respectively and expanded for two weeks. CD19CAR T-cells were co-cultured with Daudi cells at a ratio of 5:1 or co-cultured with imDCs and Daudi cells at a ratio of 5:1:1 for 20-24 hours. Flow cytometry was used to analyze CD107a expression on CD19CAR⁺ T-cells (CD3⁺).
Figure 11. T-cells were transduced with LV(Mock), LV(CD19CAR), LV(NAP-CD19CAR) respectively and expanded for two weeks. T-cells were labeled with CellTrance Violet day and co-cultured with Daudi cells at a ratio of 1:1 for 4 days. T-cells proliferation was analyzed by flow cytometry.
Figure 12. Schematic illustration of the experiments as presented in Figures 13-14.

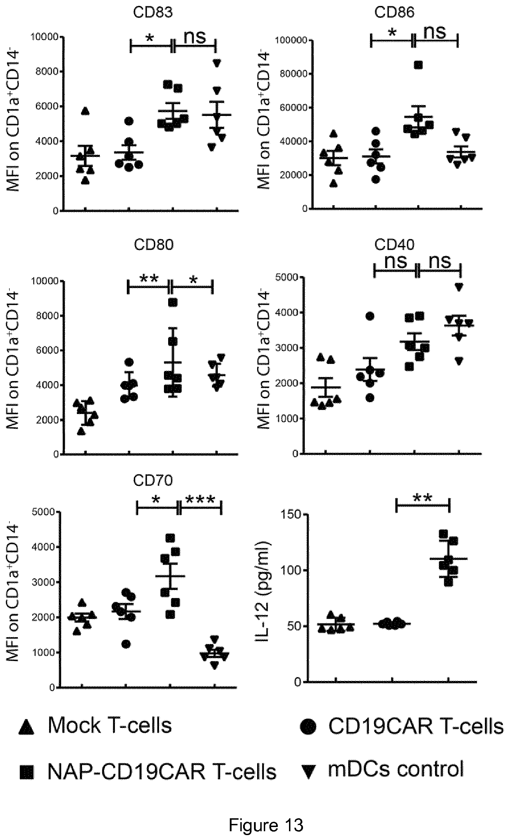

Figure 13. T-cells were transduced with LV(Mock), LV(CD19CAR), LV(NAP-CD19CAR) respectively and expanded for two weeks. The transduced and expanded T-cells were co-cultured with Daudi target cells at a ratio of 5:1 for 4 days. Supernatant was collected and directly added on monocytes-derived imDCs for 48 hours to investigate whether it could mature the imDCs. In parallel, imDCs were treated with maturation cocktail (R848, poly(I:C) and IFN-y) and used as mDC positive control in all experiments. Phenotypic analysis of DCs was assessed by flow cytometry as CD83, CD86, CD80, CD40, and CD70. Four days co-cultures were set up of transduced and expanded T-cells, Daudi target cells and autologous imDCs at ratio of 5:1:1. ELISA was used to analyze IL-12 production from supernatant collected from the co-cultures.
Figure 14. Four days co-cultures were set up of transduced and expanded T-cells, Daudi target cells and autologous imDCs at ratio of 5:1:1. Human chemokine and cytokine array was measured from supernatant collected from the co-cultures. The data was showed as heat map and related to levels detected for co-cultures of LV(Mock)-transduced T-cells.
Figure 15. Schematic illustration of the experiments presented in Figure 16.

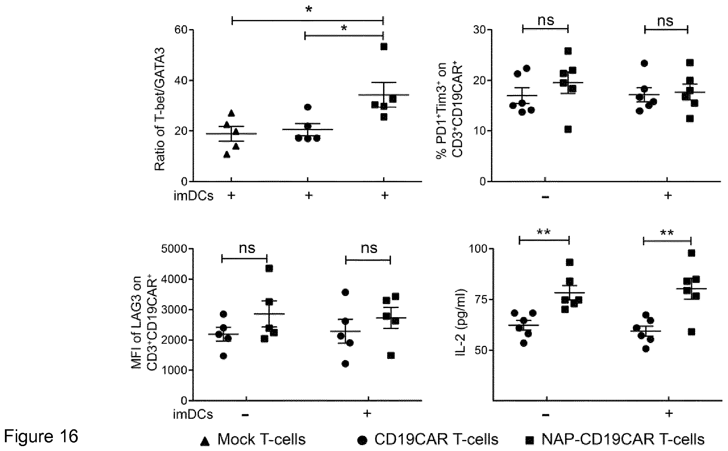

Figure 16. T-cells were transduced with LV(Mock), LV(CD19CAR), LV(NAP-CD19CAR) respectively and expanded for two weeks. Transduced and expanded T-cells were co-cultured with Daudi target cells at a ratio of 5:1 or co-cultured with Daudi target cells and imDCs at a ratio of 5:1:1 for 4 days. GATA3 and T-bet expression was assessed on CD4⁺CD3⁺ T-cells by flow cytometry. The ratio of T-bet/GATA3 (Th1/Th2) is shown. Expression of T-cell exhaustion marker PD-1 and Tim3 (double positivity) was assessed by flow cytometry. MFI of immune checkpoint receptor marker LAG3 was assessed by flow cytometry. The supernatant was collected from the co-culture and ELISA was used to analyze IL-2 production from T-cells.
Figure 17. T-cells were transduced with LV(Mock), LV(CD19CAR), LV(NAP-CD19CAR) respectively and expanded for two weeks. The supernatant saved from co-culture of T-cells and target Daudi cells at ratio 5:1 for 4 days was added to the bottom of wells and freshly isolated neutrophils, monocytes, NK cells or imDCs were seeded on the top of the membrane and placed in the wells. Migration of cells was assessed after 1.5-2 h incubation at 37°C. The percent migrating cells was measure as (RLU_{T-cells+Daudi} - RLU_{only T-cells}) / (RLU_{total migration cells}) × 100 %.
Figure 18. Schematic illustration of the experiments as presented in Figures 19-20.
Figure 19. T-cells were transduced with LV(Mock), LV(CD19CAR), LV(NAP-CD19CAR) respectively and expanded for two weeks. Transduced and expanded T-cells were co-cultured with freshly isolated autologous neutrophils for 48 hours and surface markers on the CD15⁺ neutrophils were measured by flow cytometry.
Figure 20. T-cells were transduced with LV(Mock), LV(CD19CAR), LV(NAP-CD19CAR) respectively and expanded for two weeks. Transduced and expanded T-cells were co-cultured with freshly isolated autologous neutrophils for 48 hours. ELISA was used to measure cytokines releasing from supernatant of co-culture of T-cells, neutrophils and Daudi target cells.
Figure 21. T-cells were transduced with LV(Mock), LV(CD19CAR), LV(NAP-CD19CAR) respectively and expanded for two weeks. a) Illustration of trans-well culture of monocytes (CD14⁺) (bottom) and T-cells with Daudi target cells (top). b-d) The phenotype of monocytes was measured by flow cytometry as CD14, CD1a, CD83, CD80, CD86, CD40 and CD70.
Figure 22. Schematic illustration of the retrovirus constructs used to transduce mouse T-cells.
Figure 23. Sketch of experiment schedule. Female 5-6 weeks old BALB/c mice were injected s.c with 1×10⁵ syngeneic A20 (CD19⁺) tumor cells. They were treated systemically on days 15 and 18 by i.v. injections of 5×10⁶ CD19CAR T-cells or NAP-CD19CAR T-cells and 30 IU/mL human IL-2.
Figure 24. Tumor growth for each individual mouse is presented for each group. Average tumor growth of each group is presented. The Kaplan-Meier survival curve demonstrates survival data.
Figure 25. NAP-CD19CAR T-cells exhibited more activation *in vivo.* Presence of activated T-cells was presented as % PD-1⁺CD8⁺ out of CD3⁺ draining lymph node, in spleen, and blood.
Figure 26. Mice were sacrificed and the tumors were extracted and analyzed for immune cell infiltration by flow cytometry. T-cell exhaustion in the tumors was analyzed as % PD-1⁺Tim3⁺ of CD8⁺CD3⁺ T-cells. DCs in tumor were analyzed as CD11C^{high}. Neutrophils in tumor were analyzed as Gr1+ out of CD11b⁺ cells. Each group contained at least 4 mice.

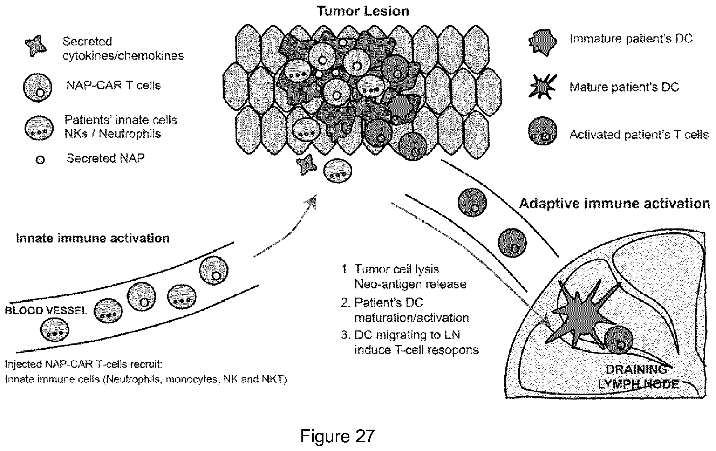

Figure 27. Illustration of the expected mechanism for NAP-CD19CAR T-cells in cancer immunotherapy. NAP expression is induced from the inducible NFAT-IL-2 promoter when CAR T-cells are activated by engagement of target cells. The expression of NAP at the tumor site leads to chemokine expression and recruitment of innate immune cells (neutrophils, monocytes and NK cells) in a locally restricted way to initiate an immune attack on tumor cells, including CAR antigen-negative tumors cells that cannot be killed by CAR T-cells. Killed tumor cells can release neo-antigens, which can be taken up by resident immature dendritic cells. NAP also induces abundant Th1 cytokine secretion (IL-12, IFN-γ, IL-1β) for DC maturation and migration to draining lymph nodes and activation of cytolytic T lymphocytes

### DETAILED DESCRIPTION

Unless defined otherwise, all technical and scientific terms used herein have the meaning commonly understood by a person skilled in the art to which the present invention belongs. The following references [11-16] provide a general definition of many of the terms used in this invention. For clarity, the following definitions are used herein.

The terms "nucleic acid sequence" or "nucleotide sequence" refer to a polymer composed of nucleotides, such as ribonucleotides, deoxyribonucleotides, related naturally occurring structural variants, and/or synthetic non-naturally occurring analogs thereof, linked via phosphodiester bonds, related naturally occurring structural variants, and/or synthetic non-naturally occurring analogs thereof. Examples of such nucleic acid or nucleotide sequences are deoxyribonucleic acid (DNA) sequences and ribonucleic acid (RNA) sequences.

The term "coding sequence" refers to a nucleic acid sequence with the properties of being able to be transcribed into either a defined sequence of nucleotides (tRNA, rRNA and mRNA) and, in the case of mRNA transcription, being further translated into a polypeptide. The coding sequence may be a gene, a cDNA or a recombinant nucleic acid sequence. Accordingly, a nucleic acid sequence encoding a polypeptide is an example of such a coding sequence.

"Promoter" is the minimal nucleic acid sequence required to direct transcription. The promoter may include elements that render the promoter-depending gene expression cell-type or tissue specifically controllable or inducible by external signals or agents.

The specificity of a chimeric antigen receptor (CAR) and/or a T-cell receptor (TCR) can be determined based on affinity and/or avidity. The affinity, represented by the equilibrium constant for the dissociation of an antigen with the CAR and/or TCR (*K*_{d}), is a measure for the binding strength between an antigenic determinant and an antigen-binding site on the CAR and/or TCR. The lesser the value of *K*_{d}, the stronger the binding strength between the antigenic determinant and the CAR and/or TCR. Alternatively, the affinity can also be expressed as the affinity constant (*K*ₐ), which is 1/*K*_{d}. As will be clear to the skilled person, affinity can be determined in a manner known per se, depending on the specific antigen of interest.

Avidity is the measure of the strength of binding between a CAR and/or TCR and the pertinent antigen. Avidity is related to both the affinity between an antigenic determinant and its antigen binding site on the CAR and/or TCR and the number of pertinent binding sites present on the CAR and/or TCR.

Generally, any *K*_{d} value greater than 10⁻⁴ M (or any *K*ₐ value lower than 10⁴ M⁻¹) is generally considered to indicate non-specific binding.

Specific binding of a CAR and/or TCR to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art.

The word "a" or "an" shall not be construed as excluding the plural, i.e. reference to e.g. the use of "a molecule" does not exclude the use of plural molecules.

The present embodiments generally relate to T-cells, also referred to as T-lymphocytes, and in particular to such T-cells useful in T-cell immunotherapy.

The T-cells of the present embodiments are useful in T-cell immunotherapy, for instance in the management of cancer. The T-cells of the embodiment are genetically modified to have a significantly improved effect in terms of, among others, enhanced cytotoxicity and enhanced immunomodulation capacity. The improvements achieved by the T-cells of the embodiments imply that the T-cells are effective in T-cell immunotherapy also for solid tumors.

A first aspect of the embodiments relates to a vector. This vector can be used to genetically modify a T-cell of the embodiments, such as in a transduction process. The vector according to the embodiments comprises a nucleic acid sequence encoding a chimeric antigen receptor (CAR) and/or a nucleic acid sequence encoding a T-cell receptor (TCR). The vector also comprises a nucleic acid sequence encoding a *Helicobacter pylori* neutrophil activating protein (HP-NAP) and/or a nucleic acid sequence encoding an immunologically equivalent fragment of HP-NAP. The immunologically equivalent fragment of the HP-NAP is a fragment including active polypeptide domains of at least 20-40 amino acid residues of the HP-NAP, preferably selected from the group consisting of SEQ ID NO: 9 to 12.

A CAR is generally designed by fusing a single chain variable fragment (scFv) from a monoclonal antibody, such as a murine monoclonal antibody, as the antigen-recognizing domain with T-cell signaling domains to achieve downstream signaling upon antigen recognition. CARs can recognize antigens in a major histocompatibility complex (MHC) independent manner. This can be advantageous since tumors can usually evade host T-cell recognition by downregulating human leukocyte antigen (HLA) (in humans, MHC is often denoted HLA) on their surface. In addition, CARs can also recognize non-protein surface molecules, such as carbohydrates and glycolipids. Thus, a CAR comprises an extra-cellular antibody-derived scFv that binds specifically to an antigen, such as a tumor-associated antigen (TAA), a transmembrane domain and an intracellular signaling domain configured to transmit an activation signal upon binding of the antigen, such as TAA, to the scFv.

CAR as used herein also encompasses functional variants and equivalents thereof. Such CAR variants have an extracellular antigen-binding domain, preferably an antibody-derived antigen-binding domain, that binds to an antigen, such as TAA. The CAR variants also have a transmembrane domain and an intracellular signaling domain configured to transmit an activation signal upon binding of an antigen to the antigen-binding domain. This activation signal, in turn, preferably induces or activates an inducible promoter that controls expression of HP-NAP or an immunologically equivalent fragment thereof as further described herein.

A TCR is a molecule found on the surface of T cells that is responsible for recognizing fragments of antigen as peptides bound to MHC molecules. When the TCR engages with an antigenic peptide and MHC, the T-cell is activated through signal transduction, that is, a series of biochemical events mediated by associated enzymes, co-receptors, specialized adaptor molecules, and activated or released transcription factors.

Non-limiting examples of TCRs that can be used include CMVpp65 TCR and TARP-TCR.

In an embodiment, the vector comprises nucleic acid sequence(s) encoding one or multiple, i.e. at least two CARs. In another embodiment, the vector comprises nucleic acid sequence(s) encoding one or multiple TCRs. In a further embodiment, the vector comprises nucleic acid sequence(s) encoding one or multiple CARs and nucleic acid sequence(s) encoding one or multiple TCRs.

In the embodiments with multiple CARs, multiple TCRs or a combination of at least one CAR and at least one TCR, the CARs, TCRs or CAR(s) and TCR(s) preferably bind specifically to different antigens, such as different TAAs.

*Helicobacter pylori* (HP) neutrophil activating protein (NAP) is a dodecameric protein that acts as a virulence factor in *H. pylori* bacterial infection. It is made of 12 monomeric subunits and each subunit is comprised of four α-helices. The surface of NAP is highly positively charged and has capacity of interacting with and activating human white blood cells (WBCs), also denoted leukocytes.

HP-NAP plays a critical role in migration of neutrophils to inflamed tissue during *H. pylori* infection. HP-NAP promotes strong binding of neutrophils and monocytes binding to endothelium and extravasation by upregulating surface expression of β2 integrin. It can also active neutrophils in producing reactive oxygen species (ROS) and myeloperoxidases. HP-NAP also activates secretion of other pro-inflammatory cytokines, such as tumor necrosis factor alpha (TNF-α) and interleukin 8 (IL-8), also referred to as chemokine (C-X-C motif) ligand 8 (CXCL8), which in turn induce adhesion molecules expression like vascular cell adhesion molecule (V-CAM), intercellular adhesion molecule (I-CAM) and secretion of IL-8 by endothelia cells. In addition, HP-NAP can also induce neutrophil secretion of several cytokines and chemokines expression, such IL-8, macrophage inflammatory protein 1 alpha (MIP-1α) and MIP-1β, also referred to as chemokine (C-C motif) ligand 4 (CCL4). These cytokines and chemokines in turn attract, by chemotaxis, neutrophils to the site of inflammation.

HP-NAP is a toll-like receptor 2 (TLR-2) agonist, is chemotactic for neutrophils, monocytes and can mature dendritic cells both *in vitro* and *in vivo.* It can also stimulate secretion of IL-12 and IL-23, which are Th-1 polarizing cytokines. HP-NAP stimulates monocytes to differentiate and mature to dendritic cells (DCs) by upregulating expression of HLA - antigen D related (HLA-DR), cluster of differentiation 80 (CD80) and CD86. It also has pivotal immunoregulatory functions in aiding cytotoxic T-cells and natural killer (NK) cells activation. HP-NAP can induce T-cells to secrete high level of interferon gamma (IFN-γ) and low level of IL-4, also suggesting a Th1 polarizing response. This is coincident with report that *H*. *pylori* infected humans indicate a strong Th1 polarizing response.

In an embodiment, the HP-NAP preferably comprises, or has, an amino acid sequence selected from any of the following sequences: 
MKTFEILKHLQADAIVLFMKVHNFHWNVKGTDFFNVHKATEEIYEEFADMFDDLAERIVQLGHHPLVTLS
EAIKLTRVKEETKTSFHSKDIFKEILEDYKYLEKEFKELSNTAEKEGDKVTVTYADDQLAKLQKSIWMLQ
AHLA (SEQ ID NO: 1)
 
MKTFEILKHLQADAIVLFMKVHNFHWNVKGTDFFNVHKATEEIYEEFADMFDDLAERIVQLGHHPLVTLS
EAIKLTRVKEETKTSFHSKDIFKEILEDYKHLEKEFKELSNTAEKEGDKVTVTYADDQLAKLQKSIWMLQ
AHLA (SEQ ID NO: 2)
 
MKTFEILKHLQADAIVLFMKVHNFHWNVKGTDFFNVHKATEEIYEEFADMFDDLAERIVQLGHHPLVTLS
EALKLTRVKEETKTSFHSKDIFKEILEDYKYLEKEFKELSNTAEKEGDKVTVTYADDQLAKLQKSIWMLQ
AHLA (SEQ ID NO: 3)
 
MKTFEILKHLQADAIVLFMKVHNFHWNVKGTDFFNVHKATEEIYEEFADMFDDLAERIVQLGHHPLVTLS
EALKLTRVKEETKTSFHSKDIFKEILEDYKHLEKEFKELSNTAEKEGDKVTVTYADDQLAKLQKSIWMLQ
AHLA (SEQ ID NO: 4)
 
MKTFEILKHLQADAIVLFMKVHNFHWNVKGTDFFNVHKATEEIYEGFADMFDDLAERIVQLGHHPLVTL
SEAIKLTRVKEETKTSFHSKDIFKEILEDYKYLEKEFKELSNTAEKEGDKVTVTYADDQLAKLQKSIWML
QAHLA (SEQ ID NO: 5)
 
MKTFEILKHLQADAIVLFMKVHNFHWNVKGTDFFNVHKATEEIYEGFADMFDDLAERIVQLGHHPLVTL
SEALKLTRVKEETKTSFHSKDIFKEILEDYKYLEKEFKELSNTAEKEGDKVTVTYADDQLAKLQKSIWML
QAHLA (SEQ ID NO: 6)
 
MKTFEILKHLQADAIVLFMKVHNFHWNVKGTDFFNVHKATEEIYEGFADMFDDLAERIVQLGHHPLVTL
SEAIKLTRVKEETKTSFHSKDIFKEILEDYKHLEKEFKELSNTAEKEGDKVTVTYADDQLAKLQKSIWML
QAHLA (SEQ ID NO: 7)
 
MKTFEILKHLQADAIVLFMKVHNFHWNVKGTDFFNVHKATEEIYEGFADMFDDLAERIVQLGHHPLVTL
SEALKLTRVKEETKTSFHSKDIFKEILEDYKHLEKEFKELSNTAEKEGDKVTVTYADDQLAKLQKSIWML
QAHLA (SEQ ID NO: 8)
 An immunologically equivalent fragment of HP-NAP is a fragment including active polypeptide domains of at least 20-40 amino acid residues of HP-NAP. Non-limiting but illustrative examples of immunologically equivalent fragments of HP-NAP include:
EILKHLQADAIVLFMKVHNFHWNVKGTDFFNVHKAT (SEQ ID NO: 9) corresponding to amino acids no. 5-40 of SEQ ID NO: 1-8
NTAEKEGDKVTVTYADDQLAKLQKSIWMLQAHLA (SEQ ID NO: 10) corresponding to amino acids no. 110-144 of SEQ ID NO: 1-8
ATEEIYEEFADMFDDLAERIVQLGHHPLVTLSEALK (SEQ ID NO: 11) corresponding to amino acids no. 39-74 of SEQ ID NO: 3-4
LTRVKEETKTSFHSKDIFKEILEDYKHLEKEFKELS (SEQ ID NO: 12) corresponding to amino acids no. 75-110 of SEQ ID NO: 3, 8

More information of immunologically equivalent fragments of HP-NAP can be found in [17], the teaching of which with regard to immunologically equivalent fragments of HP-NAP disclosed in paragraph 32, "Definition of the dominant T-cell epitopes of HP-NAP recognized by HP-NAP-specific T-cells derived from the gastric infiltrates induced by *H. pylori".*

The vector comprises, in an embodiment, nucleic acid sequence(s) encoding one or multiple HP-NAPs. In another embodiment, the vector comprises nucleic acid sequence(s) encoding one or multiple immunologically equivalent fragments of HP-NAP. In a further embodiment, the vector comprises nucleic acid sequence(s) encoding one or multiple HP-NAPs and nucleic acid sequence(s) encoding one or multiple immunologically equivalent fragments of HP-NAP.

In the case of multiple immunologically equivalent fragments of HP-NAP, at least one fragment is preferably from the N-terminal portion of HP-NAP, such as SEQ ID NO: 9 or 11, and at least one other fragment is preferably from the C-terminal portion of HP-NAP, such as SEQ ID NO: 10 or 12.

The nucleic acid sequence encoding HP-NAP or the immunologically equivalent fragment thereof may encode a fusion protein of HP-NAP or the immunologically equivalent fragment and at least one other peptide or protein. For instance, the fusion protein could be a fusion protein between HP-NAP or the immunologically equivalent fragment and a signal peptide for secretion. In such a case, the fusion protein will, when expressed in T-cells, be secreted from the T-cells due to the presence of the signal peptide. Non-limiting, but illustrative, examples of such signal peptides that can be used include Ig kappa chain V-IV region B17 signal peptide, such as the sequence M-VLQTQVFISLLLWISGAYG (SEQ ID NO: 13), leader sequence of IL-2, also referred to as IL-2 signal sequence (ss), such as the sequence MYRMQLLSCIALSLALVTNS (SEQ ID NO: 14), signal peptide sequence of CD33, such as the sequence MPLLLLLPLLWAGALA (SEQ ID NO: 15), or an artificial signal peptide MWWRLWWLLLLLLLLWPMVWA (SEQ ID NO: 16).

The vector may comprise a single nucleic acid molecule comprising nucleic acid sequence(s) encoding one or more CARs and/or one or more TCRs and nucleic acid sequence(s) encoding one or more HP-NAPs and/or one or more immunologically equivalent fragments of HP-NAP. In another embodiment, the vector comprises multiple nucleic acid molecules. In such a case, at least one of the coding sequences for CAR(s), TCR(s), HP-NAP(s) and immunologically equivalent fragment(s) of HP-NAP is present in a first nucleic acid molecule of the vector and at least one other of the coding sequences is present on at least one other nucleic acid molecule of the vector.

The vector is preferably an expression vector, i.e. a vector comprising at least one nucleic acid molecule comprising coding sequences that can be expressed, such as transcribed and translated, in a host cell, such as host T-cell, comprising the expression vector. The expression vector is in an embodiment selected among DNA molecules, RNA molecules, plasmids, episomal plasmids and virus vectors.

In an embodiment, the vector is a virus vector. In a particular embodiment, the virus vector is selected from a group consisting of a lentiviral vector, an adenoviral vector, an adeno-associated viral vector, a rertroviral vector and a hybrid vector.

Lentiviruses are a subclass of retroviruses. They are adapted as gene delivery vehicles (vectors) thanks to their ability to integrate into the genome of non-dividing cells, which is the unique feature of lentiviruses as other retroviruses can infect only dividing cells. The viral genome in the form of RNA is reverse-transcribed when the virus enters a cell, such as a T-cell, to produce DNA, which is then inserted into the genome at a position by the viral integrase enzyme. The vector, now called a provirus, remains in the genome and is passed on to the progeny of the cell if it divides. For safety reasons lentiviral vectors typically never carry the genes required for their replication. To produce a lentivirus, several plasmids are transfected into a so-called packaging cell line, commonly HEK 293. One or more plasmids, generally
referred to as packaging plasmids, encode the virion proteins, such as the capsid and the reverse transcriptase. Another plasmid contains the genetic material to be delivered by the vector. It is transcribed to produce the single-stranded RNA viral genome and is marked by the presence of the ψ (psi) sequence. This sequence is used to package the genome into the virion.

Retroviruses are one of the mainstays of current gene therapy approaches. The recombinant retroviruses, such as the Moloney murine leukemia virus, have the ability to integrate into the host genome in a stable fashion. They contain a reverse transcriptase that allows integration into the host genome. Retroviral vectors can either be replication-competent or replication-defective. Replication-defective vectors are the most common choice because the viruses have had the coding regions for the genes necessary for additional rounds of virion replication and packaging replaced with other genes, or deleted. These virus are capable of infecting T-cells and delivering their viral payload, but then fail to continue the typical lytic pathway that leads to cell lysis and death.

If the vector is a lentiviral or retroviral vector then the nucleic acid sequence(s) encoding CAR and/or TCR and HP-NAP and/or the immunological equivalent fragment of HP-NAP is/are preferably RNA sequence(s).

Adenoviral vector as used herein include adenovirus vectors and adenovirus-derived virus vectors.

Adenoviral DNA does not integrate into the genome and is not replicated during cell division. An adeno-derived virus vector is based on an adenovirus but in which various modifications have been done, such as relating to the nucleotide sequences coding replication proteins, regulation protein, viral surface proteins, etc.

Adeno-associated virus (AAV) is a small virus that infects humans and some other primate species. AAV can infect both dividing and non-dividing cells and may incorporate its genome into that of the host cell. Moreover, AAV mostly stays as episomal, performing long and stable expression. Whereas AAV packages a single strand of DNA and requires the process of second-strand synthesis, self-complementary adeno-associated virus (scAAV) packages both strands which anneal together to form double stranded DNA. By skipping second strand synthesis, scAAV allows for rapid expression in the cell.

If the vector is an adenoviral vector then the nucleic acid sequence(s) encoding CAR and/or TCR and HP-NAP and/or the immunologically equivalent fragment of HP-NAP is/are preferably DNA sequence(s).

A hybrid vector is a vector virus that is genetically engineered to have qualities of more than one vector. For instance, a hybrid vector may be a combination of an adenovirus and a lentivirus.

In a particular embodiment, the vector is lentiviral vector. In another particular embodiment, the vector is a self-inactivating (SIN) lentiviral vector.

In an embodiment, the nucleic acid sequence encoding HP-NAP and/or the nucleic acid sequence encoding the immunologically equivalent fragment of HP-NAP is under transcriptional control of an inducible promoter. According to this embodiment, inducible expression of HP-NAP and/or the immunologically equivalent fragment thereof is preferred over constitutive expression in terms of reducing or minimizing any unwanted toxicity that may occur when using a constitutive promoter for controlling transcription of HP-NAP and/or the immunologically equivalent fragment thereof.

Thus, transducing T-cells with a vector with constitutive expression of HP-NAP and/or the immunologically equivalent fragment thereof could cause unwanted toxicity effects to the T-cells. Experimental data as presented herein, indicates that inducible expression of HP-NAP did not negatively affect T-cell function.

In an embodiment, the inducible promoter is activated upon binding of an antigen, preferably a TAA, to the CAR and/or TCR. Thus, binding of the antigen to the extracellular domain of CAR and/or TCR activates the inducible promoter via the intracellular signaling domain of CAR and/or TCR.

A non-limiting but illustrative example of such an inducible promoter is N nuclear factor of activated T-cells (NFAT) binding motifs followed by the minimal human IL-2 promoter. In this example N is 1-10, preferably 4-8 and more preferably 6. Thus, in a preferred embodiment, the nucleic acid sequence encoding HP-NAP and/or the immunologically equivalent fragment thereof is under transcriptional control of an inducible NFAT-IL-2 promoter. The NFAT-IL-2 promoter preferably comprises, or more preferably consists of, six repeats of NFAT element and the minimal human IL-2 promoter. This promoter and is selective and inducible by CAR/TCR - antigen engagement.

Although, an inducible promoter is preferred in some applications as indicated above, the embodiments are not limited thereto. Thus, the nucleic acid sequence encoding HP-NAP and/or the nucleic acid sequence encoding the immunologically equivalent fragment of HP-NAP could be under transcriptional control of a constitutive promoter. In a particular embodiment, the constitutive promoter is selected from a group consisting of the human elongation factor 1 alpha (EF1a) promoter, the cytomegalovirus (CMV) promoter and the CAG promoter, preferably the EF1a promoter.

In an embodiment, the nucleic acid sequence(s) encoding CAR and/or TCR is(are) under transcriptional control of a promoter selected from a group consisting of the human EF1a promoter, the CMV promoter and the CAG promoter, preferably the EF1a promoter.

In an embodiment, the vector comprises a nucleic acid sequence encoding CAR having an extra-cellular antibody-derived scFv that binds specifically to B-lymphocyte antigen CD19 (CD19) and under transcriptional control of the EF1a promoter. The vector also comprises a nucleic acid sequence encoding HP-NAP with a signal peptide for secretion and under transcriptional control of an inducible NFAT-IL-2 promoter. In a particular embodiment, the vector is a lentiviral vector, such as a SIN lentiviral vector. The vector preferably comprises a nucleic acid molecule comprising the EF1a promoter, the nucleic acid sequence encoding CAR, the inducible NFAT-IL-2 promoter and the nucleic acid sequence encoding HP-NAP.

Another aspect of the embodiments relates to a T-cell. The T-cell comprises a CAR, a TCR, a nucleic acid sequence encoding a CAR and/or a nucleic acid sequence encoding a TCR. The T-cell also comprises HP-NAP, an immunologically equivalent fragment thereof, a nucleic acid sequence encoding HP-NAP and/or a nucleic acid sequence encoding an immunologically equivalent fragment of HP-NAP. The immunologically equivalent fragment of the HP-NAP is a fragment including active polypeptide domains of at least 20-40 amino acid residues of the HP-NAP, preferably selected from the group consisting of SEQ ID NO: 9 to 12.

The nucleic acid sequence(s) encoding CAR, TCR, HP-NAP or the immunologically equivalent fragment of HP-NAP can be transcribed in the T-cell to produce the CAR protein, TCR protein, HP-NAP protein or the immunologically equivalent fragment of HP-NAP in the T-cell.

The CAR and/or TCR of the T-cell binds specifically to an antigen, preferably a TAA. In an embodiment, the TAA is selected from a group consisting of CD19, CD20, mucin 1 (MUC1), mesothelin (MSLN), NY-ESO-1, alpha-fetoprotein (AFP), carcinoembryonic antigen (CEA), human epidermal growth factor receptor 2 (HER2), tumor protein p53 (p53), Ras protein (RAS), melanoma-associated antigen (MAGE), preferably CD19.

CD19 is expressed by and present on B-cells. Since CD19 is a hallmark of B-cells, the protein has been used to diagnose cancers that arise from this type of cell - notably B-cell lymphomas. CD19 has also been implicated in autoimmune diseases and may be a useful treatment target.

CD20 is expressed on all stages of B cell development except the first and last; it is present from late pro-B-cells through memory cells, but not on either early pro-B-cells or plasma blasts and plasma cells. It is found on B-cell lymphomas, hairy cell leukemia, B-cell chronic lymphocytic leukemia, and melanoma cancer stem cells. CD20 is a target in several diseases including B-cell lymphomas and leukemias, such as chronic lymphocytic leukemia, systemic lupus erythematosus (SLE), myalgic encephalomyelitis, follicular lymphoma, rheumatoid arthritis, multiple sclerosis, for non-Hodgkin's lymphoma and immune thrombocytopenia.

Mesothelin is a 40 kDa protein present on normal mesothelial cells and overexpressed in several human tumors, including mesothelioma and ovarian and pancreatic adenocarcinoma.

NY-ESO-1 is a human tumor antigen of the cancer/testis family. It is highly expressed in many poor-prognosis melanomas.

AFP is the most abundant plasma protein found in the human fetus. AFP is elevated in various diseases, including omphalocele, hepatocellular carcinoma/hepatoma, neural tube defects, nonseminomatous germ cell tumors, yolk sac tumor, ataxia telangiectasia, hepatocellular carcinoma, germ cell tumors and metastatic cancers of the liver.

CEA is normally produced in gastrointestinal tissue during fetal development, but the production stops before birth. Therefore, CEA is usually present only at very low levels in the blood of healthy adults. However, the serum levels are raised in some types of cancer, such as colon carcinoma, gastric carcinoma, pancreatic carcinoma, lung carcinoma, breast carcinoma, and medullary thyroid carcinoma, as well as some non-neoplastic conditions like ulcerative colitis, pancreatitis, cirrhosis, COPD, Crohn's disease and hypothyroidism.

HER2 is a member of the human epidermal growth factor receptor family. Amplification or over-expression of this oncogene has been shown to play an important role in the development and progression of breast cancer. Over-expression is also known to occur in ovarian cancer, gastric cancer, salivary duct carcinomas, causes adenocarcinoma of the lung and aggressive forms of uterine cancer, such as uterine serous endometrial carcinoma.

p53 is important in multicellular organisms, where it prevents cancer formation, thus, functions as a tumor suppressor. The *TP53* gene is the most frequently mutated gene in human cancer. If the TP53 gene is damaged, tumor suppression is severely compromised.

Mutations in the RAS are very common, being found in 20 % to 30 % of all human tumors. Activating mutations of the Ras protein and upstream elements of the Ras protein may play a role in more than two-thirds of all human cancers, including most metastatic disease.

The MAGE gene family is typically completely silent in normal adult tissues, with the exception of male germ cells and, for some of them, placenta. By contrast, these MAGE genes are expressed in various kinds of tumors.

In an embodiment, HP-NAP is expressed and secreted by the T-cell upon binding of an antigen, preferably a TAA, to the CAR and/or the TCR. In such an embodiment, the nucleic acid sequence encoding HP-NAP and preferably also encodes a signal peptide for secretion. The nucleic acid sequence encoding HP-NAP and/or the immunologically equivalent fragment of HP-NAP is preferably codon optimized for expression in human T-cells.

In an embodiment, the T-cell comprises the nucleic acid sequence encoding HP-NAP and/or the nucleic acid sequence encoding the immunologically equivalent fragment of HP-NAP under transcriptional control of an inducible promoter.

In an embodiment, the inducible promoter is activated upon binding of an antigen, preferably a TAA, to CAR and/or TCR. In a particular embodiment, the inducible promoter is *N* NFAT binding motifs followed by the minimal human IL-2 promoter, N is 1-10, preferably 4-8, more preferably 6.

In another embodiment, the T-cell comprises the nucleic acid sequence encoding HP-NAP and/or the nucleic acid sequence encoding the immunologically equivalent fragment of HP-NAP under transcriptional control of a constitutive promoter, such as the EF1a promoter, the CMV promoter or the CAG promoter.

In an embodiment, the T-cell comprises the nucleic acid sequence encoding CAR and/or the nucleic acid sequence encoding TCR under transcriptional control of a promoter selected from a group consisting of the EF1a promoter, the CMV promoter and the CAG promoter, preferably the EF1a promoter.

The nucleic acid sequences encoding CAR, TCR, HP-NAP or the immunologically equivalent fragment of HP-NAP may be present in the genome of the T-cell. Alternatively, at least some of the nucleic acid sequences may be present in one or more episomal nucleic acid molecules, i.e. not present in the chromosomes of the T-cell.

In an embodiment, the T-cell according to the second aspect comprises the vector according to the first aspect previously described herein. The vector could be integrated into the genome of the T-cell or be a non-integrated extrachromosomal vector.

The present technology takes a new approach in T-cell immunotherapy by switching the immunosuppressive tumor immune microenvironment (TME) to a more pro-inflammatory TME by local expression of the HP-NAP protein upon CAR/TCR T-cells recognition of target cells. Experimental data showed that inducible expression of HP-NAP improved CAR T-cell cytotoxicity in the presence of autologous DCs, that it attracted innate immune cells and increased secretion of pro-inflammatory cytokines and chemokines, which enhanced CAR T-cells function *in vitro.*

HP-NAP, when introduced into and produced by CAR T-cells, lead to maturation and activation of DCs, promoted secretion of Th1 polarizing cytokine IL-12 and acted as a chemoattractant for immune cells, all of which can positively modulate the TME and enhance the CAR T-cells function.

To control expression of HP-NAP locally in the tumor and avoid systemic toxicity, HP-NAP was, in an embodiment, engineered to be expressed by the inducible NFAT-IL-2 promoter, which was activated only upon antigen recognition by CAR T-cells both *in vitro* and *in vivo.*

NAP-CD19CAR T-cells, i.e. T-cells transduced by a lentiviral vector comprising the sequence encoding for CD19CAR under control of the EF1a promoter and the HP-NAP coding sequence under control of the inducible NFAT-IL-2 promoter, have enhanced cytotoxicity, when co-cultured with autologous imDCs *in vitro.* NAP-induced maturation and activation of DCs, caused the DCs to express T-cell co-stimulatory molecules like CD80/86 and CD40 and secrete IL-12, which can in turn help T-cell priming and stimulation.

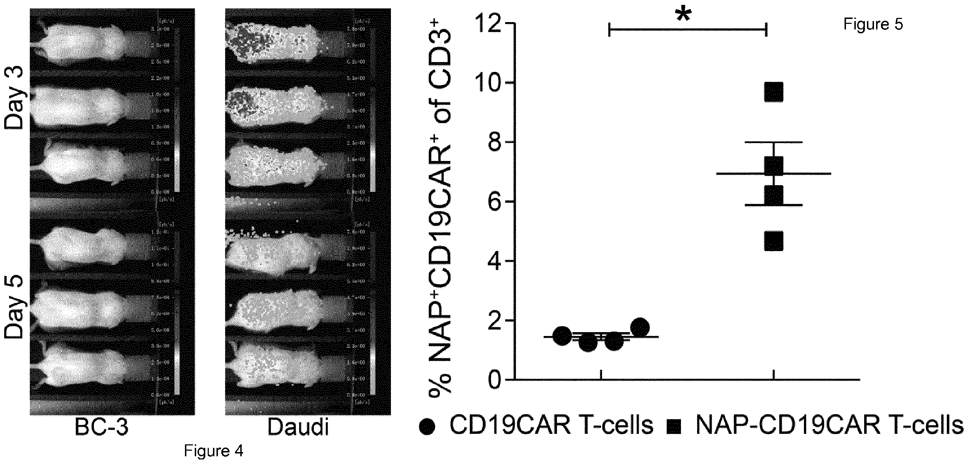

DCs in the TME have an impaired function and are usually associated with immunosuppression. However, local expression of HP-NAP by activated CAR T-cells according to the embodiments could help reverse immune suppression, and prime bystander tumor specific T-cells apart from aiding the infused CAR T-cells. Additionally, we observed an increased expression of CD70 on DCs when co-cultured with NAP-CD19CAR T-cells (Figure 4). CD70 primes effective CD8 T-cells response by licensed DCs. It binds CD27 on CD8 T-cells and improves T-cells viability, persistence and resistance to oxidative stress.

T-cell exhaustion is a primary factor limiting the antitumor efficacy of CAR T-cells *in vivo,* and T-cell exhaustion is mainly attributed to chronic antigenic stimulation of T-cells. NAP-CD19CAR T-cells had increased cytotoxic capacity when co-cultured with DCs, but importantly HP-NAP did not lead to increased exhaustion of T-cells in terms of PD1/Tim3 double expression, LAG3 expression, and IL-2 secretion *in vitro.* Thus, HP-NAP did not negatively affect T-cell function.

HP-NAP is not only Th1 polarizing but is also known to revert Th2 polarity of helper T-cells to Th1 type helper cells. NAP-CD19CAR T-cells had a higher ratio of T-bet:GATA3 (Th1:Th2 type transcription factor expression) and higher expression of IFN-γ and IL-2 when co-cultured with autologous DCs and antigen-positive target cells. HP-NAP also induced Th1 polarization on bystander T-cells that are not CAR-antigen specific. T-cells present in the TME are usually anergy, so rescuing their activity is important to mount an efficient anti-tumor immune response.

Generally, one of the key limiting factor for efficient immunotherapy of solid tumors is lack of infiltrating immune cells. The T-cells of the embodiment capable of local HP-NAP secretion promoted infiltration of neutrophils but also acted as a chemoattractant for monocytes, NK cells and imDC. We also detected important chemokines like CCL-1, CCI-2, MIP-1α/β in supernatants from HP-NAP activated CAR T-cell:DC:target cell co-cultures. These chemokines can further promote infiltration of pro-inflammatory immune cells in the TME. For instance, CCL-1 can attract monocytes, NK cells and DCs, CCL-2 can recruit monocytes, memory T-cells and DCs, while MIP-1α (CCL-3) and MIP-1β (CCL-4) are chemoattractants for NK cells, and monocytes, thus promoting an influx of immune cell infiltration to the tumor.

Thus, experimental data as presented herein indicates that NAP-CD19CAR T-cells, i.e. T-cells transduced by a lentiviral vector comprising the sequence encoding for CD19CAR under control of the EF1a promoter and the HP-NAP coding sequence under control of the inducible NFAT-IL-2 promoter, cultured together with immature dendritic cells (imDCs), displayed higher cytotoxity of CD19⁺ target cells than did control CD19CAR T-cells. HP-NAP secreted by the NAP-CD19CAR T-cells matured the co-cultured imDCs and stimulated them to secrete key cytokines, such as IL-12, IL-1α/β, and chemokines, such as CXCL11, CXCL 12. In addition, the HP-NAP secreted from the NAP-CD19CAR T-cells also induced recruitment of monocytes, neutrophils and NK cells.

The improvement achieved in the NAP-CD19CAR T-cells did not negatively affect T-cell function and did not result in any T-cells exhaustion. In addition, NAP-CD19CAR T-cells induced Th1 polarization in the presence of imDCs.

A third aspect of the embodiment thereby relates to a pharmaceutical composition comprising a T-cell according the embodiments and a dendritic cell, preferably an immature dendritic cell.

The T-cells and the DCs of the pharmaceutical composition may be of the same source, such as autologous T-cells and autologous DCs to be administered to a same patient from which they have previously been isolated. It may also be possible to use autologous T-cells and allogenic DCs, allogenic T-cells and autologous DCs or allogenic T-cells and allogenic DCs in the pharmaceutical composition. In the case of allogenic T-cells and/or DCs, the cells are isolated from another individual of the same species as the current patient to which the pharmaceutical composition is to be administered.

The pharmaceutical composition may additionally comprise one or more pharmaceutically acceptable carriers, vehicles and/or excipients. Non-limiting examples of such pharmaceutically acceptable carriers, vehicles and excipients include injection solutions, such as saline or buffered injection solutions.

Further aspects of the embodiments relates to a T-cell according to the embodiments or a pharmaceutical composition according to the embodiments for use as a medicament, for use in T-cell immunotherapy or for use in treating, reducing and/or preventing cancer. In the latter aspect, the cancer may, in an embodiment, be a hematological cancer. However, the improvements to the T-cells of the embodiments imply that they can have positive effects in treating, reducing and/or preventing solid tumors.

The T-cells and pharmaceutical composition of the embodiments may also be used to prevent, reduce and/or treat neoplasm, such as benign neoplasm, *in situ* neoplasm, malignant neoplasm, hyperplasia, and warts.

The T-cells of the embodiments or the pharmaceutical composition of the embodiments can be used in combination with other therapies traditionally used in T-cell immunotherapy or cancer therapy. For instance, the T-cells or the pharmaceutical composition can be combined with chemotherapy, surgery and/or radiotherapy treatment in the case of cancer diseases.

The pharmaceutical composition preferable comprises an effective amount of the mentioned T-cells and DCs. As used herein, "effective amount" indicates an amount effective, at dosages and for periods of time necessary to achieve a desired result. For example, in the context of inhibiting a tumor growth an effective amount is an amount that, for example, induces, remission, reduces tumor burden, and/or prevents tumor spread or growth compared to the response obtained without administration of the cells. Effective amounts may vary according to factors, such as the disease state, age, sex, weight of the patient.

"Treating" or "treatment" as used herein and is well understood in the art, means an approach for obtaining beneficial or desired results, including clinical results. Beneficial or desired clinical results could include, for instance, alleviation or amelioration of one or more symptoms or conditions, diminishment of extent of disease, stabilized state of disease, i.e. prevent worsening, preventing spread of disease, delay or slowing of disease progression, amelioration or palliation of the disease state, diminishment of the reoccurrence of disease, and remission. "Treating" or "treatment" may also prolong survival as compared to expected survival if not receiving any treatment.

"Preventing" or "prophylaxis" as used herein and is well understood in the art, means an approach in which a risk of developing a disease or condition is reduced or prevented, including prolonging or delaying disease development. For instance, a patient predisposed to develop a disease, such as due to genetic or hereditary predisposition, could benefit for administration of the T-cells or pharmaceutical composition of the embodiments to prevent, reduce the risk of, delaying and/or slowing development of the disease.

The patient is preferably a human patient. The embodiments may, however, also be applied in veterinary applications, i.e. non-human patients, such as non-human mammals including, for instance, primates, monkeys, apes, cattle, sheep, pigs, goats, horses, cats, dogs, mice, rats and guinea pigs.

The T-cells or the pharmaceutical composition may be administered to the patient according to various routes including, for instance, intravenous, subcutaneous, intraperitoneal, intramuscular or intratumoral administration.

The present embodiments also relate to a method of inducing maturation of immature dendritic cells. The method comprises contacting, preferably *in vitro,* the immature dendritic cells with a T-cell according to the embodiments.

### EXAMPLES

Chimeric antigen receptor (CAR)-engineered T cells, targeting the pan B-cell marker CD19, have made remarkable progress in clinical studies for both acute and chronic B-cell leukemias. The results observed for B-cell lymphomas have been poorer than for leukemias. The reason may be that the semisolid structure of lymphomas leading to penetration obstacles and an immune suppression microenvironment within these tumors. In order to improve CAR T-cell therapy for lymphomas, CD19CAR T-cells have been engineered with *Helicobacter pylori* Neutrophil-Activating Protein (NAP) that is expressed and secreted upon CAR recognition of CD19 antigen positive tumor cells. Experimental results show that NAP-CD19CAR T-cells, cultured together with autologous immature dendritic cells (DCs), displayed higher cytotoxicity of CD19⁺ target cells *in vitro,* than did conventional CD19CAR T-cells. NAP secreted by the activated NAP-CD19CAR T-cells matured the co-cultured DCs and stimulated them to secret key cytokines (Interleukin 12 (IL-12), IL-1α/β) and chemokines (C-X-C motif chemokine 11 (CXCL11), CXCL12). In addition, the secreted NAP also induced recruitment of monocytes, neutrophils and natural killer (NK) cells.

### Materials and Methods

### Primary cell isolation and generation and cell line cultures

Peripheral blood mononuclear cells (PBMCs) were isolated using Ficoll-Paque separation (GE Healthcare Life Science, Uppsala, Sweden) from fresh buffy coats obtained from the Blood center at the Uppsala University Hospital. PBMCs were cultured in RPMI-1640 medium supplemented with 10 % heat-inactivated foetal bovine serum (FBS), 1 mM sodium pyruvate, 100 IU penicillin/mL and 100 µg streptomycin/mL (1 % PeSt).

Human monocyte-derived DCs were obtain by CD14⁺ beads isolation (Miltenyi Biotec, Bergisch Gladbach, Germany) from PBMCs and differentiated over 5 days into immature DCs (imDCs) with 50 ng/mL granulocyte-macrophage colony-stimulating factor (GM-CSF) (Gentaur, Brussels, Belgium,) and 25 ng/mL IL-4 (Gentaur). Fresh medium supplemented with cytokines was replaced every two days. Human T-cells were also cultured with the same medium as PBMCs. The human B-cell lymphoblast cell line Daudi (CD19⁺, ATCC CCL-213) were cultured in RPMI-1640 supplemented with 10 % heat-inactivated FBS and 1 % PeSt. BC-3 (CD19-, ATCC CRL-2277) were cultured in RPMI-1640 supplemented with 20 % heat-inactivated FBS and 1 % PeSt. All media supplements and culture media were purchased from Invitrogen (Carlsbad, CA), unless mentioned elsewhere. All cells were cultured in a humidified incubator with a 5 % CO₂ atmosphere at 37°C.

### Lentivirus construction and production

The sequence of CD19CAR was designed as described previously [8]. The sequence encoding for CD19CAR was cloned into a third generation self-inactivating (SIN) lentiviral vector (SBI, System Biosciences, Mountain View, CA) under control of the elongation factor-1 alpha (EF1a) promoter to construct LV(CD19CAR). The human codon optimized NAP transgene sequence tagged with an artificial signal peptide for secretion was placed under the control of an inducible NFAT-IL-2 promoter designed as described previously [9]. The NFAT-IL-2 promoter consisted of 6 repeats of nuclear factor of activated T-cells (NFAT) element and the minimal human IL-2 promoter. The NAP sequence along with the NFAT-IL-2 promoter was cloned into lentiviral vector to construct LV(NAP-CD19CAR). The NAP gene was replaced with the sequence coding for nano-luciferase to construct LV(nanoLuc-CD19CAR). The LV(Mock) was made with an empty lentiviral vector. All the sequences were purchased from GeneScript (Piscataway, NJ).

### Flow cytometry analyses

All antibodies for fluorescence-activated cell sorting (FACS) staining were purchased from Biolegend, San Diego, CA. The stained cells were analyzed in BD CANTOII flow cytometer (BD Biosciences).

### T-cell engineering, expansion and validation of NAP expression

PBMCs (5×10⁶ cells) were activated with OKT-3 (100 ng/mL, BioLegend, San Diego, CA) provided at day 1 and IL-2 (100 IU/mL, Proleukin, Novartis, Basel, Switzerland) provided at day 2 in culture medium for 3 days. Activated T-cells (1×10⁶ cells) were re-suspended in 2×10⁷ IU concentrated lentivirus together with 10 mg/ml protamine sulfate (Sigma-Aldrich, St Louis, Mo) and IL-2 (100 IU) in a volume of 20 µL and incubated for 4 hours. The T-cells were transduced again the day after in the same manner and cultured in 1 ml culture medium supplied with IL-2 (100 IU/mL) for 7 days before transgene expression was measured. T-cells were then expanded using the rapid expansion protocol (REP) as previously described [10]. NAP expression was assessed after co-culturing NAP-CD19CAR T-cells with Daudi (CD19⁺) at 5:1 ratio and blocking NAP secretion by adding Brefeldin A (BD GolgiPlug^{™}, Frankline Lakes, NJ) for 12 h followed by intercellular staining with a primary α-NAP antibody (kind gift from Dr. Marina de Bernard, University of Padova, Italy) and a secondary Alexa Fluor 488 rabbit anti-mouse IgG antibody (Invitrogen). The CD19CAR expression was verified by staining with an allophycocyanin-conjugated (APC-conjugated) rabbit anti-mouse Ig(H+L) Fab fragment (Invitrogen). T-cells were identified by staining with Pacific blue-conjugated anti-human CD3 antibody.

### Nano-luciferase expression from T-cells in vitro and in vivo

LV(nanoLuc-CD19CAR) transduced T-cells were generated and expanded as described above. Nano-luciferase expression was measured in nanoLuc-CD19CAR T-cells and nanoLuc-CD19CAR T-cells co-cultured with Daudi target cells at ratio 5:1 for 24 h. Nano-luciferase expression was measured by directly adding substrate from Nano-Glo^{®} Luciferase Assay kit (Promega, Madison, WI) to the cells. Female, 5 weeks old, NOD-SCID mice (Janvier Labs, France) were injected intravenously with 1×10⁷ nanoLuc-CD19CAR T-cells together with Daudi (CD19⁺) target cells or BC-3 (CD19-) target cells (3 mice per group) at a ratio 2:1 in a total volume of 200 µL on day 0. Nano-luciferase expression was measured by intravenous injection of substrate (Nano-Glo^{®} Luciferase Assay, Promega) on day 3, day 5 and monitored by NightOWL *in vivo* Imaging system (Berthold Technologies GmbH & Co. Kg, Germany).

### T-cell cytotoxicity assay

T-cells were rested for 3 days after REP expansion in low dose IL-2 (20 lU/mL) before they were used for functional assays. Monocytes were separated from PBMCs by CD14⁺ magnetic beads isolation (Miltenyi Biotec). The monocytes were differentiated into immature DCs (imDCs) by culturing them in DC culture medium supplemented with 20 ng/mL human IL-4 (Gentaur, Brussels, Belgium) and 100 ng/mL GM-CSF (Gentaur) for 5 days. Lentivirus-transduced T-cells were co-cultured with firefly luciferase-tagged target cells (Daudi) and with or without autologous imDCs at ratios 5:1:1 for 4 days in a total volume of 100 µL. The luciferase expression and activity (as indicator of viability of target cell) was measured as mentioned previously [10]. The relative cell viability was calculated by normalizing relative light unit (RLU) from co-cultured samples against the RLU from only target cells.

### Cytokine secretion, surface marker expression and T-cell proliferation

Transduced T-cells were co-cultured with Daudi target cells at ratio of 1:1 or co-cultured with Daudi target cells and imDCs at a ratio of 5:1:1 for 20-24 hours. Interferon gamma (IFN-γ) and IL-2 secretion from T-cells and IL-12 secretion from DCs was measured in the supernatants from the co-cultures by enzyme-linked immunosorbent assay (ELISA) (Mabtech, Nacka Strand, Sweden). T-cells were stained for expression of the degranulation marker CD107a using a FITC-CD107 antibody (Biolegend). Transduced T-cells were co-cultured with Daudi target cells at a ratio of 5:1 for 4 days and were stained for expression of the exhaustion markers by using FITC-PD1, PE-Tim3 and FITC-LAG3 antibodies.

In another experiment transduced T-cells were stained with CellTracer Violet dye (Invitrogen) and cultured with Daudi target cells at ratio of 1:1 for 4 days. T-cells were then analyzed by flow cytometry to measure the dilution of the violet dye as an indication of cell proliferation.

### DCs maturation phenotype analyses and cytokine array

Transduced T-cells were co-cultured with Daudi target cells at ratio of 5:1 for 4 days to stimulate NAP expression. Supernatant was collected and directly added on imDCs for 48 hours.

Phenotypic analysis of DCs was performed using the following fluorophore-labeled mouse monoclonal antibodies (maturation DC phonotype panel): PECy7-CD14, BV510-CD1a, APC-CD83, PE-CD80, BV421-CD86, FITC-CD40 and APC-Cy7-CD70 and along with matched mouse IgG isotype controls. imDCs were also maturated for 24 hours in culture medium containing 1000 IU/ml IFN-γ (Shenandoah Biotechnology, Warwick, PA), 20 µg/ml polyinosinic:polycytidylic acid (poly(I:C), Sigma-Aldrich) and 2.5 µg/ml R848 (InvivoGen, San Diego, CA) and used as positive control for DC maturation (mDC). The supernatant collected from co-cultures of T-cells, autologous imDCs and Daudi target cells at ratio 5:1:1 for 4 days were analyzed for the presence of cytokines using Proteome ProfilerTM Human Cytokine Array Kit, Panel A (R&D Systems, Inc., Minneapolis, MN).

### Human monocytes trans-well culture with T-cell with target cells

CD14⁺ monocytes were isolated from PBMCs by using anti-CD14⁺ beads (Miltenyi Biotec). Monocytes were kept in the bottom of 24-well plate and trans-well inserts with 0.45 µm pore size membrane (Corning) containing T-cells together with Daudi cells (5:1 ratio) and cultured for 4 days. Phenotypic analysis of monocytes, caused by the supernatant from T-cells and target cells, was performed using maturation DC phonotype panel as described above.

### Migration assay

Cell migration was assessed using a 96-well ChemoTx^{®} Disposable Chemotaxis System (Neuro Probe, Gaithersburg, MD) containing a polycarbonate membrane filter (3 µm pore size). The supernatant saved from co-culture of T-cells with Daudi target cells at ratio 5:1 for 4 days were placed on the bottom and 2×10⁵ freshly isolated neutrophils, CD14⁺ monocytes, CD56⁺ NK cells and imDCs differentiated from human monocytes were seeded on the top of the trans-well membrane. Migration of cells was assessed after 1.5-2 h incubation at 37°C and was quantified by CellTiter-Glo^{®} Luminescent Cell Viability Assay (Promega). The percent migration of cells was measure as (RLU_{T-cells+Daudi} - RLU_{only T-cells}) / (RLU_{total migration cells}) × 100 %.

### Multicolor FACS staining of Th1/Th2

T-cells were transduced with virus and after 7 days culturing, co-cultured with autologous imDCs and Daudi target cells at ratio 5:1:1 for 4 days. T-cells were stained with surface markers: Pacific blue-CD3, Percp-CD4, and FITC-CD8. T-cells were also permeabilized by using True-Nuclear Transcription Factor Buffer Set (Biolegend) for 1 hour. The permeabilized T-cells were then stained with PE-Cy7-T-bet and PE-GATA3. The ratio of Tbet / GATA3 was calculated for the CD3⁺CD4⁺ T cell population.

### Neutrophils activation assay

Transduced T-cells were rested for 3 days after expansion in low dose IL-2 (20 lU/mL) and then co-cultured with freshly isolated autologous neutrophils and Daudi target cells at ratio 5:1:1 for 48 hours in a total volume of 100 µL. The neutrophils were gated as CD15⁺ cells and activation marker was analyzed by staining with following fluoresced labeled antibodies: PECy7-CD11b, PE-HLA-DR, Pacific Blue-CD54 and APC-CD66b. The cytokines (IL-12, IL-1β, IFN-γ and IL-2) were measured from co-culture by ELISA (Mabtech).

### In vivo animal experiment

Female 5-6 weeks old BALB/c mice (The Janvier Labs) were injected s.c. into the hind flank with 1×10⁵ A20 cells in dPBS (Invitrogen) on day 0. After tumor appearance on days 12 and 15, the mice received i.v. injections of 5×10⁶ T-cells in the presence of 30 IU/mL human IL-2. On day 16 post-tumor inoculation mice were sacrificed and cells from tumors, spleen, blood and lymph node were collected and enzymatically digested into single-cell suspensions. Cells were stained with: Pe/Cy7-PD-1, AF647-Tim3, FITC-CD3, PerCP-CD4, BV421-CD8, BV510-CD45 in order to evaluate exhaustion of T-cells in tumor; APC/Cy7-B220, PE/Cy7-Gr1, BV421-Ly6C, FITC-Ly6G, PE-CD11c, PerCP-CD11b in order to evaluate the tumor microenvironment. All antibodies were purchased from Biolegend. Tumors were measured with caliper and the tumor size was calculated as the formula: Volume = (length) × (width)² × π/6. Mice were sacrificed when the tumor volume exceeded 1 cm³.

### Statistical analysis

The data are reported as means and standard error of the mean (SEM). Statistical analysis was performed by GraphPad prism software version 6.01 (La Jolla, CA, USA). Statistical analyses in all figures were performed using multiple comparison of parametric ANOVA test (more than 2 data group) and non-parametric T Tests if only 2 data group. Values with P<0.05 were considered to be statistically significant.

### Result

### The inducible NFAT-IL-2 promoter introduced in CAR T-cells was specifically activated upon recognition of target cells both in vitro and in vivo

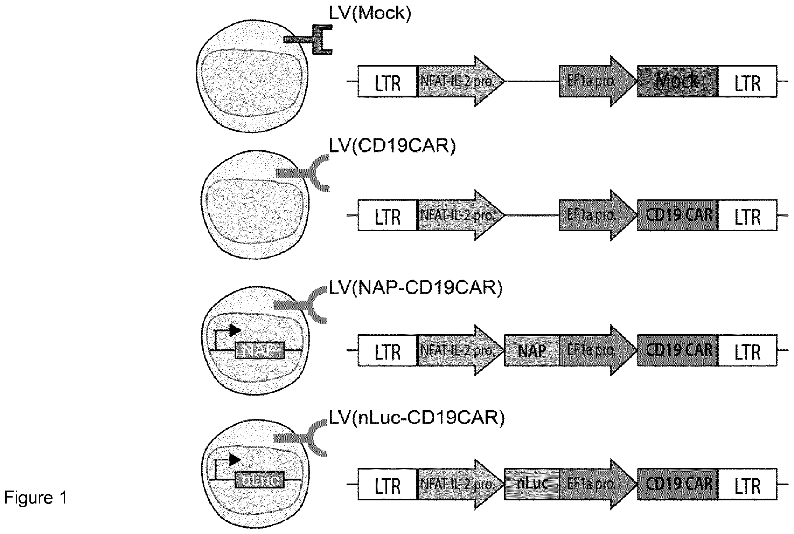

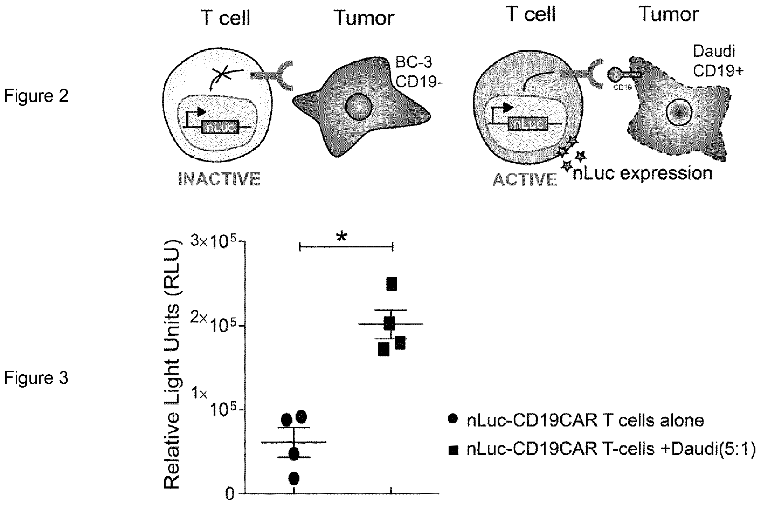

To minimize unwanted toxicity caused by constitutive expression of NAP, a lentiviral vector with an inducible recombinant promoter was constructed. It consisted of six repeats of NFAT-binding motifs followed by the minimal human IL-2 promoter. The recombinant promoter drives reporter gene expression in T-cells activated upon TCR engagement with target antigen. To validate the promoter in our system, a lentiviral vector NFAT-IL-2 promoter controlled nano-luciferase was constructed (Figure 1) and illustrated (Figure 2). The vector also contained the CD19CAR under transcriptional control of the human elongation factor 1 alpha (EF1a) promoter.

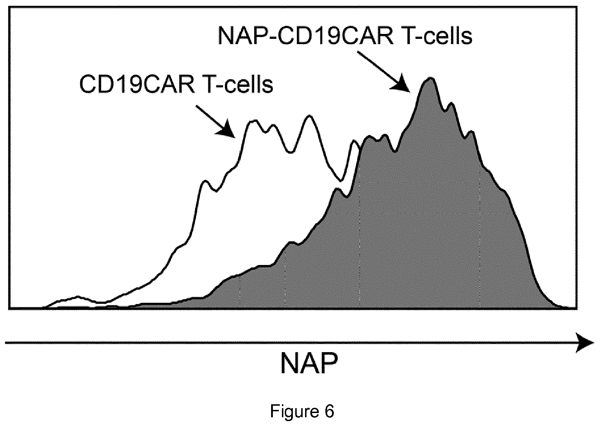

NanoLuc-CD19CAR T-cells expressed the nano-luciferase reporter gene only when co-cultured with CD19⁺ Daudi target cells (Figure 3). The activation of the inducible promoter was also evaluated *in vivo* by co-injecting nanoLuc-CD19CAR T-cells with either CD19- BC-3 cells or CD19⁺ Daudi cells. Strong luciferase signal was detected only in mice co-injected with CD19⁺ Daudi cells 3 days after injection with fading signals 5 days after injection (Figure 4). Signals were not detected when mice were co-injected with CD19- BC-3 cells (Figure 4). These data suggests that the NFAT-IL-2 promoter was selective and inducible by CAR-antigen engagement. We also verified specific NAP expression in T-cells engineered with LV(NAP-CD19CAR) lentiviral vector compared to CD19CAR lentiviral vector upon Daudi target cell stimulation (Figures 5 and 6).

### Expression of NAP improved CAR T-cell cytotoxicity in the presence of autologous immature DCs without affecting T-cell function

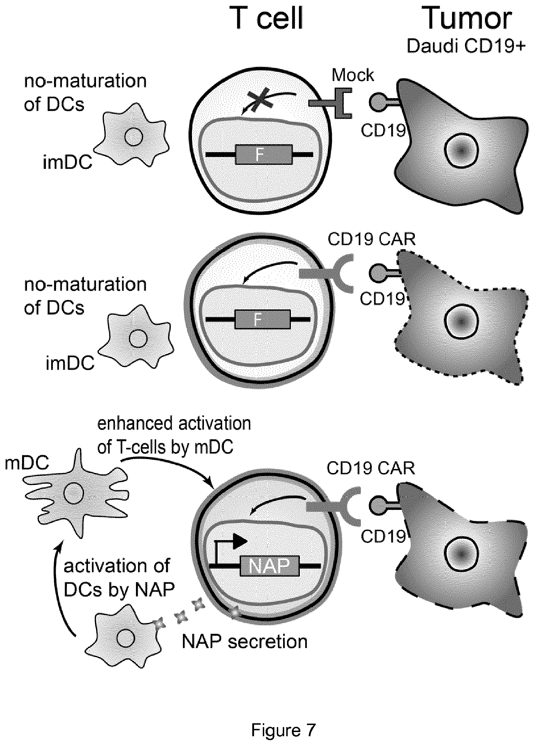

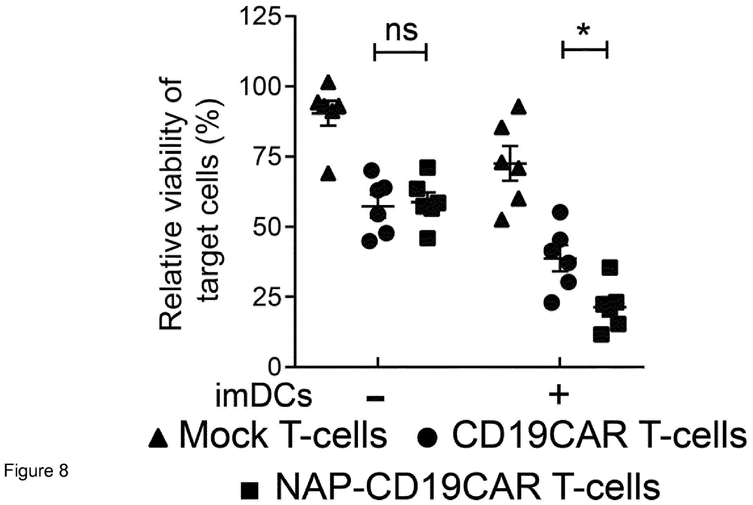

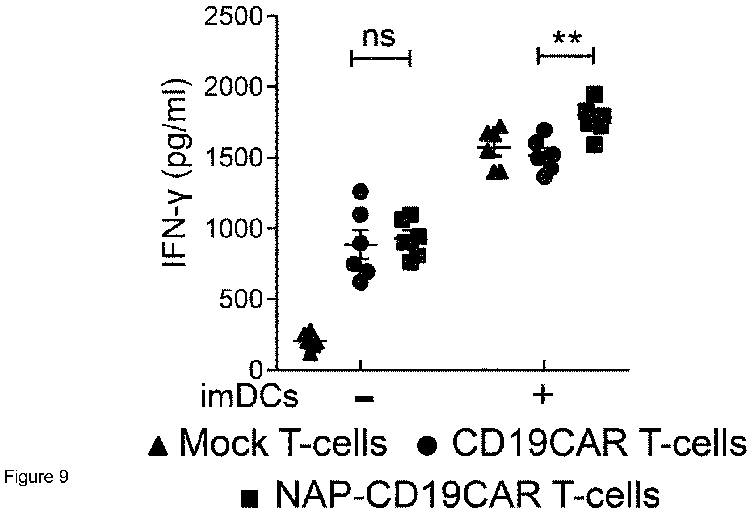

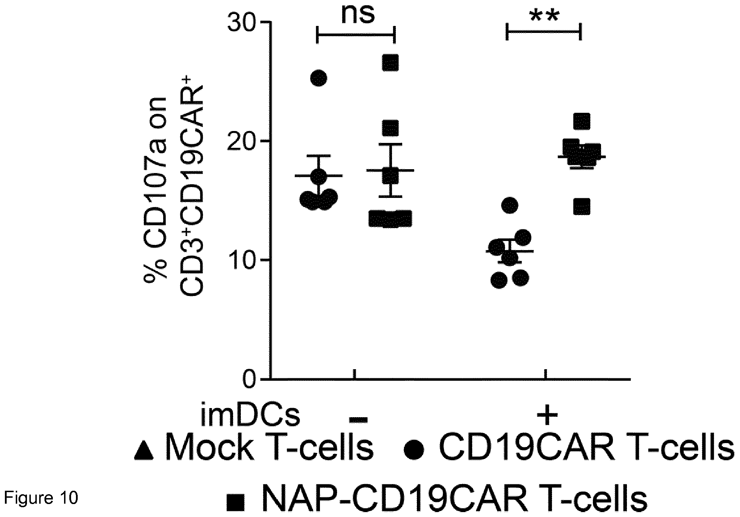

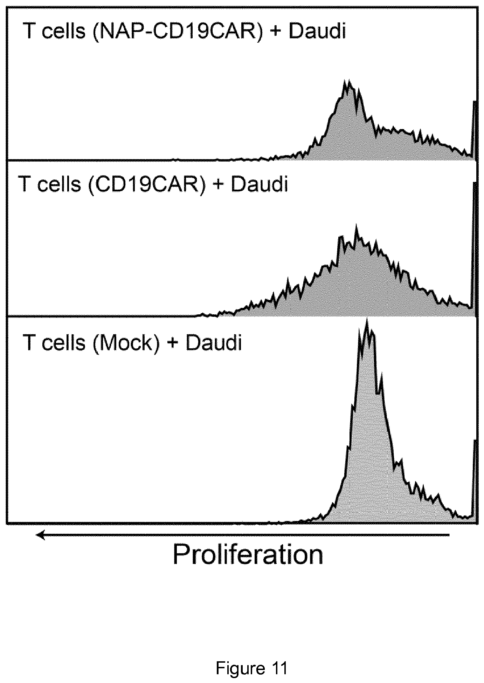

The potential toxicity of the NAP protein to CD19CAR T-cells was evaluated to confirm that NAP did not affect T-cell function negatively. Function of transduced T-cells upon target cell recognition was analyzed based on cytotoxicity to CD19⁺ target cells, IFN-γ release, surface expression of CD107 and proliferation ability as illustrated in Figure 7. T-cells transduced with LV(CD19CAR) or LV(NAP-CD19CAR) exhibited similar cytotoxicity to target cells. However, when co-cultured with autologous immature DCs (imDCs), NAP-CD19CAR T-cells displayed significantly better cytotoxicity than CD19CAR T-cells (Figure 8). There was no difference in terms of IFN-γ secretion and CD107a expression between CD19CAR T-cells and NAP-CD19CAR T-cells, while in the presence of autologous imDCs, NAPCD19CAR T-cells secreted significantly more IFN-γ and exhibited a higher degree of degranulation (CD107a expression) than CD19CAR T-cells (Figures 9 and 10). In addition, T-cells transduced with LV(CD19CAR) or LV(NAP-CD1C9AR) exhibited similar T-cell proliferation ability upon target cell recognition (Figure 11).

### NAP secreted from activated NAP-CD19CAR T-cells stimulated chemokine and cytokines secretion and promoted DC maturation

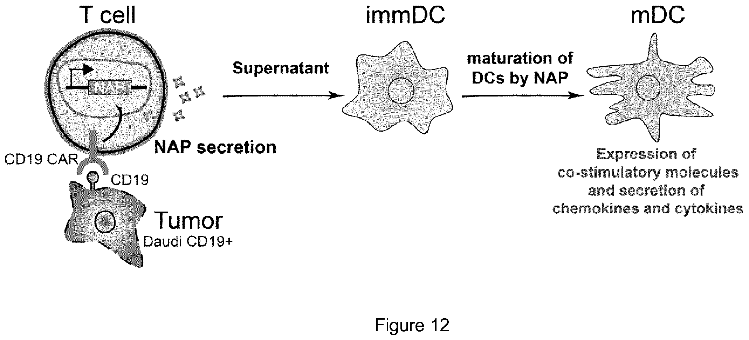

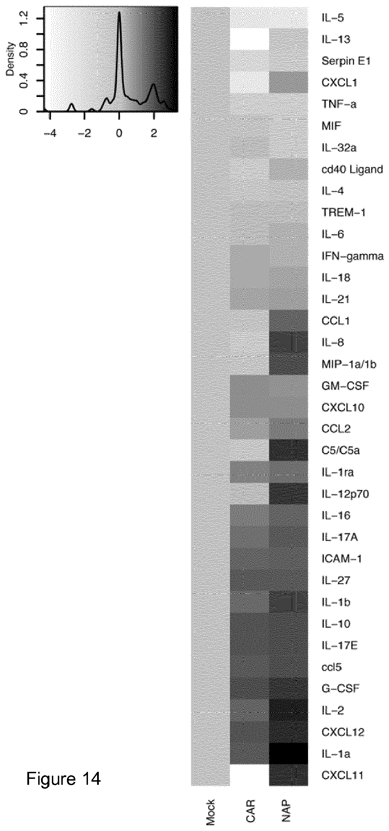

As we observed enhanced cytotoxicity displayed by NAP-CD19CAR T-cells when co-cultured with imDCs, it motivated us to further investigate the impact of NAP secretion on monocyte-derived imDCs. Supernatant collected form co-cultures of target cells and either Mock T-cells, NAP-CD19CAR T-cells or CD19CAR T-cells were examined for DC maturation capacity as illustrated in Figure 12. imDCs incubated with supernatants from NAP-CD19CAR T-cells co-cultures expressed higher levels of maturation marker CD83 as compared to imDCs incubated with supernatant from either Mock T-cells or CD19CAR T-cells (Figure 13). Strikingly, NAP matured imDCs had similar maturation and activation phenotype as DCs matured with a maturation cocktail consisting of IFN-γ, poly I:C and R848 (positive control). Furthermore, we also profiled the chemokines and cytokines present in supernatants from T-cells:imDCs:Daudi co-cultures, as NAP has been reported with Th1 polarizing property. A heat map is represented with clustered profiling data that normalized to Mock T-cells (Figure 14). Supernatants from NAP-CD19CAR T-cells co-cultures had higher amounts of innate immune cells chemo-attractants CCL-1, CCL-2, IL-8; Th1-activating cytokines IL-12, IL-16, IL-1α/β and neutrophils activating cytokines IL-8, MIP-1α/β than in supernatants obtained from CD19CAR T-cells co-cultures (Figure 14). The presence of the key Th1 polarizing cytokine IL-12 in supernatants was also confirmed and quantified by ELISA (Figure 13).

### NAP did not promote T-cells exhaustion, but induced Th1 polarized in the presence of autologous imDCs

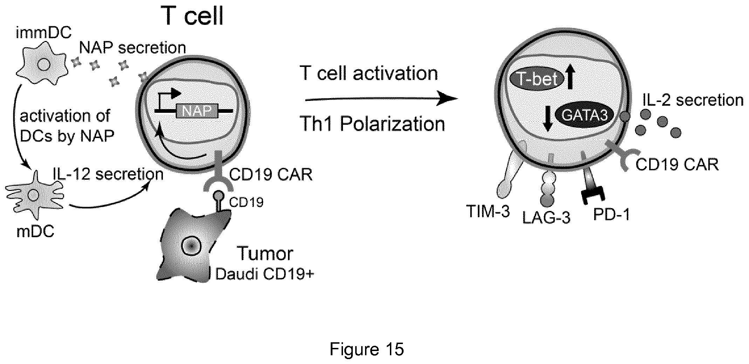

The phenotype of activated Mock T-cells, CD19CAR T-cells and NAP-CD19CAR T-cells in the presence of autologous imDCs was determined by the transcription factors T-bet and Gata-3. The differentiation of naive T-helper cells toward Th1 or Th2 cells is tightly controlled by the transcription factors T-box expressed in T-cells (T-bet) and GATA-binding protein-3 (GATA-3). The expression of T-bet and GATA3 was checked by multicolor flow cytometry (Figure 15). In the presence of imDCs, GATA3⁺ cells were slightly decreased while T-bet⁺ cells were slightly increased on NAP-CD19CAR T-cells compared to the ones on CD19CAR T-cells, leading to that the ratio of T-bet/GATA3 on CD3⁺CD4⁺-cells, which is an indication of Th1/Th2 polarization, was significantly higher in NAP-CD19CAR T-cells than that in CD19CAR T-cells (Figure 16). This result confirmed the Th1-polarization property of NAP and is consistent with the cytokine and chemokine profile in Figure 13.

High surface expression of inhibitory receptors on T-cells, such as PD-1, Tim3 and LAG3 is a hallmark of T-cell exhaustion and inhibitory markers, which is a primary factor limiting the antitumor efficacy of CAR T-cells. Induced NAP expression did not promote T-cell exhaustion as observed through evaluating the PD1⁺Tim3⁺ (double positive) CAR T-cell population (Figure 16) and CAR T-cell expression of LAG3 (Figure 16). Exhausted T-cells reduce the IL-2 production, indicating T-cells dysfunction. We found that NAP expression by T-cells increased IL-2 secretion both with and without autologous imDCs, as secretion was significantly higher than for CD19CAR T-cells (Figure 16). Together, the data shows that the presence of autologous imDCs did not affect NAPCD19CAR T-cells exhaustion and its ability to secrete IL-2.

### Innate immune cells were recruited and activated by NAP expressed from activated NAP-CD19CAR T-cells

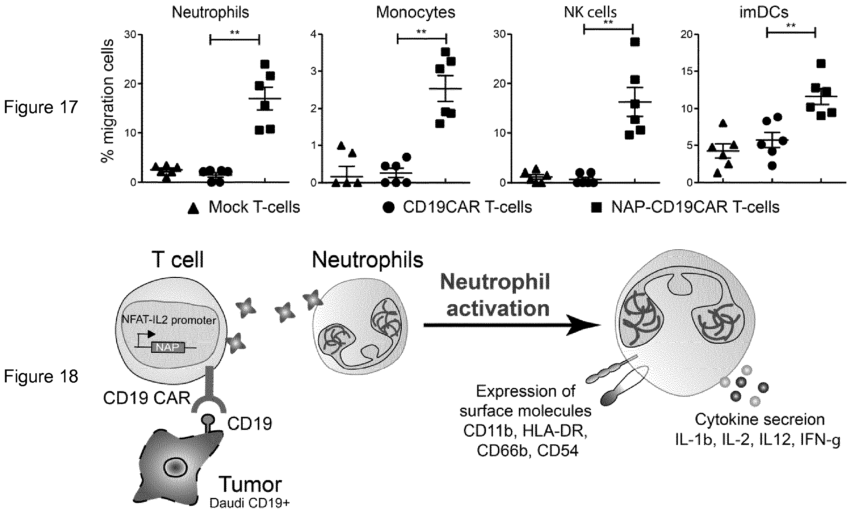

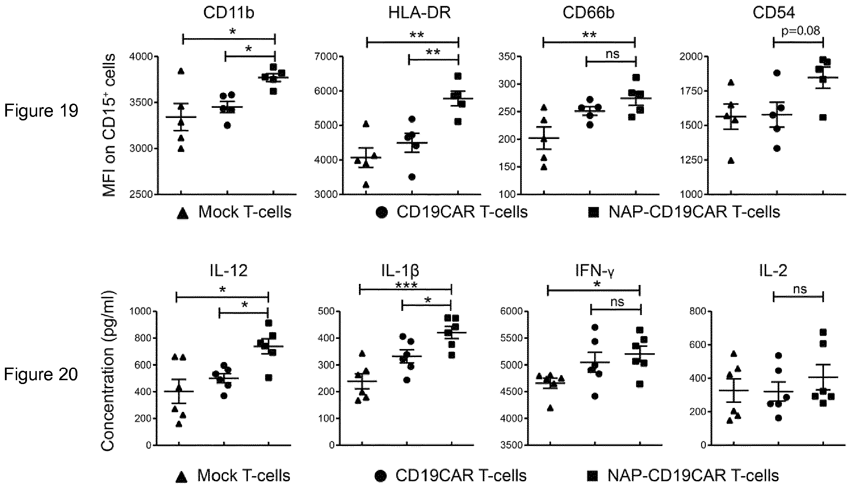

NAP is a potent chemo-attractant for neutrophils and monocytes. We also observed that supernatants from NAP-CD19CAR T-cells co-cultures were chemoattractant for neutrophils and monocytes (Figure 17). In addition, we also observed that NAP can attract NK cells and imDCs (Figure 17). Activation of neutrophils (CD15⁺) was monitored when co-cultured together with Mock T-cells, CD19CAR T-cells and NAP-CD19CAR T-cells and the Daudi target cells (Neutrophils:T-cells:Daudi) (Figure 18). Activated CD11b and HLA-DR expression on neutrophils was significantly higher in the co-culture of NAP-CD19CAR T-cells than the one of CD19CAR T-cells (Figure 19). There was also an increased expression of CD66b and CD54 expression on neutrophils in the NAP-CD19CAR T-cells co-culture (Figure 19). The activation relied on the expressed NAP upon antigen engagement (T-cells:Daudi), since neither Mock T-cell co-culture nor tumor cell alone elevated the activation markers expression. Neutrophils can produce numerous cytokines after appropriate stimulation. IL-12 and proinflammatroy cytokine IL-1β secretion were significantly higher when neutrophils were activated by NAP (Figure 20). IFN-γ and IL-2 secretion from NAP-CD19CAR T-cells co-culture were also slightly higher compared to secretion from co-cultures with CD19CAR T-cells (Figure 20).

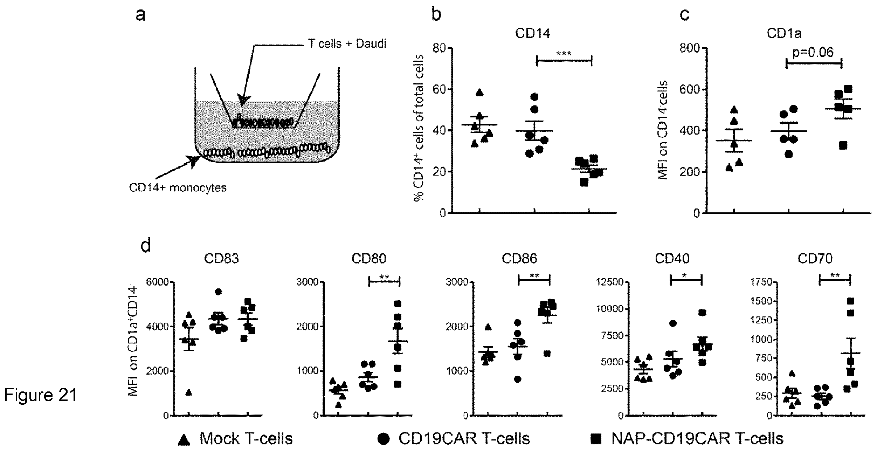

We also investigated the effect of NAP on monocyte differentiation in a trans-well co-culture assay as illustrated in Figure 21a. After 5 days trans-well co-culture between T-cell and target cells, the monocytes displayed a more differentiated, DC like phenotype (CD14-CD1a^{high}) (Figures 21b-21c). These DC-like cells were further evaluated for their maturation status. Monocytes differentiated from the NAP-CD19CAR T-cells:Daudi supernatant exhibited a more activated and matured DC phenotype with higher levels of co-stimulatory molecules including CD80, CD86, CD40 and CD70 while CD83 was expressed at similar levels as for monocytes differentiated from the CD19CAR T-cells:Daudi supernatant (Figure 21d).

### The use of NAP-CAR T-cells for therapeutic purposes

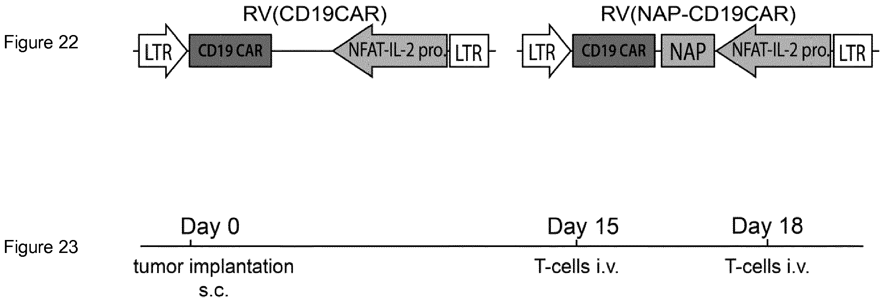

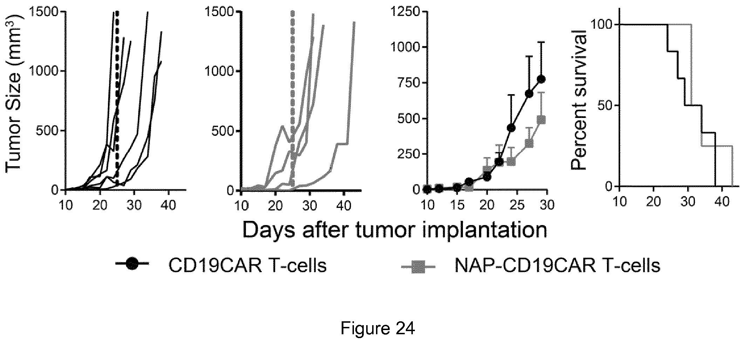

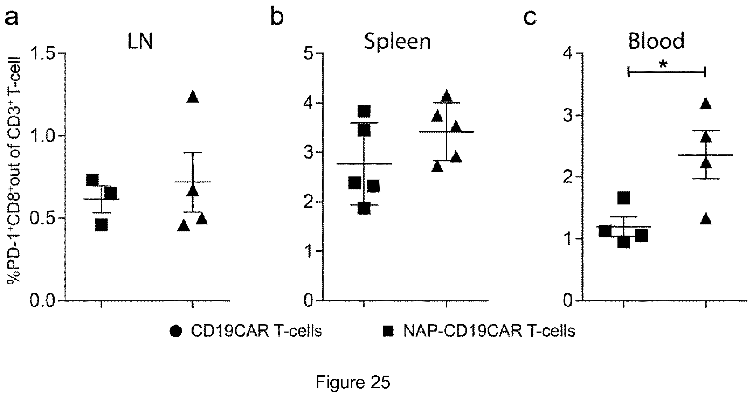

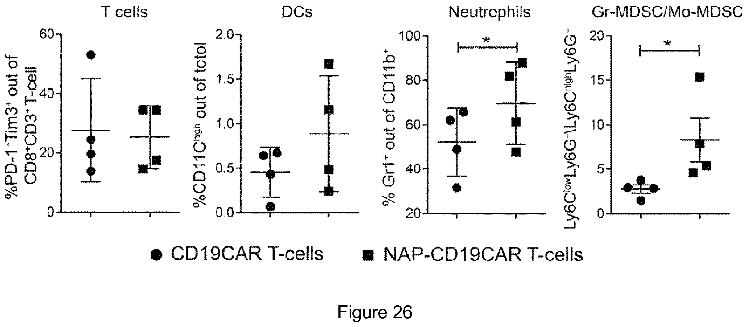

As indicated above, NAP secreted from T-cells has multiple effects on immune cells, therefore it is of important to examine the therapeutic efficacy also *in vivo.* The retrovirus constructions used *in vivo* and the experiment timeline are illustrated in Figures 22 and 23. Mice having subcutaneous lymphoma treated with NAP-CD19CAR T-cells had somewhat delayed tumor growth (Figure 24) and slightly prolonged survival of mice (Figure 24) compared to mice treated with CD19CAR T-cells. The amount of activated CD8⁺ T-cells (PD1⁺) was somewhat higher in draining lymph node, spleen and significantly higher in blood when i.v. injection with NAP-CD19CAR T-cells where used compared to CD19CAR T-cells (Figure 25). The immune cells in tumor microenvironment were also examined from resected tumors by flow cytometry. infiltrating T-cells from the NAP-CD19CAR T-cells treated group showed the same level of exhaustion as T-cells from the CD19CAR T-cells group (Figure 26). Higher amount of DCs (Figure 26) and neutrophils (Figure 26) were detected in the tumor microenvironment. When analyzing myeloid-derived suppressor cells (MDSCs) population in the tumors, we found that the ratio between granulocytic (Gr)-MDSC and monocytic (Mo)-MDSCs was higher in the NAP-CD19CAR T-cells treated group (Figure 26) indicating less monocytic suppressive MDSCs in the tumor microenvironment. An overall mechanism of NAP-T cell in a therapeutic setting is briefly sketched in Figure 27.

From the results, we propose a hypothesis for the mechanism behind NAP-CAR T-cells mode of action (Figure 27). NAP seems to be a promising immunomodulating factor to be introduced into CAR T-cells in order to alter the TME and boost anti-tumor activity.

### REFERENCES

1. Rosenberg, S.A., et al., Adoptive cell transfer: a clinical path to effective cancer immunotherapy. Nat Rev Cancer, 2008. 8(4): p. 299-308.
2. Grupp, S.A., et al., Chimeric antigen receptor-modified T cells for acute lymphoid leukemia. N Engl J Med, 2013. 368(16): p. 1509-18.
3. Morgan, R.A., et al., Cancer regression in patients after transfer of genetically engineered lymphocytes. Science, 2006. 314(5796): p. 126-9.
4. Porter, D.L., et al., Chimeric antigen receptor-modified T cells in chronic lymphoid leukemia. N Engl J Med, 2011. 365(8): p. 725-33.
5. Savoldo, B., et al., CD28 costimulation improves expansion and persistence of chimeric antigen receptor-modified T cells in lymphoma patients. J Clin Invest, 2011. 121(5): p. 1822-6.
6. Kakarla, S. and Gottschalk, S., CAR T cells for solid tumors: armed and ready to go?. Cancer J, 2014. 20(2): p. 151-5.
7. Klebnoff, C.A., et al., Prospects for gene-engineered T cell immunotherapy for solid cancers. Nat Med, 2016. 22(1): p. 26-36.
8. Milone, M.C., et al., Chimeric receptors containing CD137 signal transduction domains mediate enhanced survival of T cells and increased antileukemic efficacy in vivo. Mol Ther, 2009. 17(8): p. 1453-64.
9. Fiering, S., et al., Single cell assay of a transcription factor reveals a threshold in transcription activated by signals emanating from the T-cell antigen receptor. Genes Dev, 1990. 4(10): p. 1823-34.
10. Jin, C., et al., Allogeneic lymphocyte-licensed DCs expand T cells with improved antitumor activity and resistance to oxidative stress and immunosuppressive factors. Mol Ther Methods Clin Dev, 2014. 1: p. 14001.
11. Singleton et al., Dictionary of microbiology and molecular biology. 3rd ed, Revised, 2007, ISBN: 9780470035450.
12. Walker, The Cambridge dictionary of science and technology. 1990, ISBN: 9780521394413.
13. Rieger et al., Glossary of Genetics: Classical and Molecular. 5th ed., 1991, ISBN: 9783540520542.
14. Hale, HarperCollins dictionary of biology. 1991, ISBN: 9780064610155.
15. Lewin, Gene IX. 2007, ISBN: 9780763740634.
16. Knipe et al., Field's Virology. 5th ed., 2007, ISBN: 9780781760607.
17. EP 1 767 214 B1

## Claims

1. A vector comprising:
a nucleic acid sequence encoding a chimeric antigen receptor (CAR) and/or a nucleic acid sequence encoding a T-cell receptor (TCR); and
a nucleic acid sequence encoding a *Helicobacter pylori* neutrophil activating protein (HP-NAP) and/or a nucleic acid sequence encoding an immunologically equivalent fragment of said HP-NAP, wherein said immunologically equivalent fragment of said HP-NAP is a fragment including active polypeptide domains of at least 20-40 amino acid residues of said HP-NAP, preferably selected from the group consisting of SEQ ID NO: 9 to 12.

2. The vector according to claim 1, wherein said vector is a virus vector, preferably selected from a group consisting of a hybrid vector, a lentiviral vector, an adenoviral vector, an adeno-associated viral vector and a retroviral vector, more preferably a lentiviral vector.

3. The vector according to claim 1 or 2, wherein said nucleic acid sequence encoding said HP-NAP and/or said nucleic acid sequence encoding said immunologically equivalent fragment of said HP-NAP is under transcriptional control of an inducible promoter, preferably activated upon binding of an antigen, preferably a tumor associated antigen (TAA), to said CAR and/or said TCR, and more preferably being *N* nuclear factor of activated T-cells (NFAT) binding motifs followed by the minimal human interleukin 2 (IL-2) promoter, *N* is 1-10, preferably 4-8, more preferably 6.

4. The vector according to any of the claims 1 to 3, wherein said nucleic acid sequence encoding said CAR and/or said nucleic acid sequence encoding said TCR is under transcriptional control of a promoter selected from a group consisting of the human elongation factor 1 alpha (EF1a) promoter, the cytomegalovirus (CMV) promoter and the CAG promoter, preferably the EF1a promoter.

5. The vector according to any of the claims 1 to 4, comprising:
a nucleic acid sequence encoding said CAR having an extra-cellular antibody-derived single chain variable fragment (scFv) that binds specifically to B-lymphocyte antigen CD19 (CD19) and under transcriptional control of the human elongation factor 1 alpha (EF1a) promoter; and
a nucleic acid sequence encoding said HP-NAP with an artificial signal peptide for secretion and under transcriptional control of an inducible NFAT-IL-2 promoter comprising six repeats of nuclear factor of activated T-cells (NFAT) binding motifs.

6. A T-cell comprising:
i) a chimeric antigen receptor (CAR), a T-cell receptor (TCR), a nucleic acid sequence encoding said CAR and/or a nucleic acid sequence encoding said TCR; and
a *Helicobacter pylori* neutrophil activating protein (HP-NAP), an immunologically equivalent fragment of said HP-NAP, a nucleic acid sequence encoding said HP-NAP and/or a nucleic acid sequence encoding said immunologically equivalent fragment of said HP-NAP, wherein said immunologically equivalent fragment of said HP-NAP is a fragment including active polypeptide domains of at least 20-40 amino acid residues of said HP-NAP, preferably selected from the group consisting of SEQ ID NO: 9 to 12.

7. The T-cell according to claim 6, wherein said CAR and/or said TCR binds specifically to an antigen, preferably a tumor-associated antigen (TAA), and more preferably selected from a group consisting of B-lymphocyte antigen CD19 (CD19), CD20, mucin 1 (MUC1), mesothelin (MSLN), NY-ESO-1, alpha-fetoprotein (AFP), carcinoembryonic antigen (CEA), human epidermal growth factor receptor 2 (HER2), tumor protein p53 (p53), Ras protein (RAS), melanoma-associated antigen (MAGE), preferably CD19.

8. The T-cell according to claim 6 or 7, wherein said HP-NAP is expressed and secreted by said T-cell upon binding of an antigen, preferably a tumor-associated antigen (TAA), to said CAR and/or said TCR.

9. The T-cell according to any of the claims 6 to 8, wherein said T-cell comprises ii) said nucleic acid sequence encoding said HP-NAP and/or said nucleic acid sequence encoding said immunologically equivalent fragment of said HP-NAP under transcriptional control of an inducible promoter, preferably activated upon binding of an antigen, preferably a tumor associated antigen (TAA), to said CAR and/or said TCR, and more preferably being N nuclear factor of activated T-cells (NFAT) binding motifs followed by the minimal human interleukin 2 (IL-2) promoter, *N* is 1-10, preferably 4-8, more preferably 6.

10. The T-cell according to any of the claims 6 to 9, wherein said T-cell comprises i) said nucleic acid sequence encoding said CAR and/or said nucleic acid sequence encoding said TCR under transcriptional control of a promoter selected from a group consisting of the human elongation factor 1 alpha (EF1a) promoter, the cytomegalovirus (CMV) promoter and the CAG promoter, preferably the EF1a promoter.

11. A pharmaceutical composition comprising:
a T-cell according to any of the claims 6 to 10; and
a dendritic cell, preferably an immature dendritic cell.

12. A T-cell according to any of the claims 6 to 10 or a pharmaceutical composition according to claim 11 for use as a medicament.

13. A T-cell according to any of the claims 6 to 10 or a pharmaceutical composition according to claim 11 for use in T-cell immunotherapy.

14. A T-cell according to any of the claims 6 to 10 or a pharmaceutical composition according to claim 11 for use in treating, reducing and/or preventing cancer, preferably a solid tumor cancer.

15. A method of inducing maturation of immature dendritic cells, said method comprising contacting *in vitro* said immature dendritic cells with a T-cell according to any of the claims 6 to 10.

## Patentansprüche

1. Vektor, umfassend:
eine Nucleinsäuresequenz, die für einen chimären Antigenrezeptor (CAR) codiert, und/oder eine Nucleinsäuresequenz, die für einen T-Zell-Rezeptor (TCR) codiert; und
eine Nucleinsäuresequenz, die für ein *Helicobacter-pylori-*Neutrophil-aktivierendes Protein (HP-NAP) codiert, und/oder eine Nucleinsäuresequenz, die für ein immunologisch äquivalentes Fragment des HP-NAP codiert, wobei das immunologisch äquivalente Fragment des HP-NAP ein Fragment ist, das aktive Polypeptiddomänen von mindestens 20-40 Aminosäureresten des HP-NAP einschließt, die bevorzugt aus der Gruppe ausgewählt sind, bestehend aus SEQ ID NO: 9 bis 12.

2. Vektor nach Anspruch 1, wobei der Vektor ein Virusvektor ist, der bevorzugt aus einer Gruppe ausgewählt ist, bestehend aus einem Hybridvektor, einem lentiviralen Vektor, einem adenoviralen Vektor, einem Adeno-assoziierten viralen Vektor und einem retroviralen Vektor, stärker bevorzugt ein lentiviraler Vektor.

3. Vektor nach Anspruch 1 oder 2, wobei die Nucleinsäuresequenz, die für das HP-NAP codiert, und/oder die Nucleinsäuresequenz, die für das immunologisch äquivalente Fragment des HP-NAP codiert, unter der Transkriptionskontrolle eines induzierbaren Promotors steht, der bevorzugt bei Bindung eines Antigens, bevorzugt eines Tumor-assoziierten Antigens (TAA), an den CAR und/oder den TCR aktiviert wird und bei dem es sich stärker bevorzugt um *N* Bindemotive des Kernfaktors aktivierter T-Zellen (NFAT) handelt, gefolgt vom minimalen Promotor des humanen Interleukin-2 (IL-2), wobei *N* 1-10 ist, bevorzugt 4-8, stärker bevorzugt 6.

4. Vektor nach einem der Ansprüche 1 bis 3, wobei die Nucleinsäuresequenz, die für den CAR codiert, und/oder die Nucleinsäuresequenz, die für den TCR codiert, unter der Transkriptionskontrolle eines Promotors steht, der aus einer Gruppe ausgewählt ist, bestehend aus dem Promotor des humanen Elongationsfaktors 1 alpha (EF1a), dem Promotor des Cytomegalovirus (CMV) und dem CAG-Promotor, bevorzugt des EF1a-Promotors.

5. Vektor nach einem der Ansprüche 1 bis 4, umfassend:
eine Nucleinsäuresequenz, die für den CAR codiert, mit einem extrazellulären Antikörper-abgeleiteten einkettigen variablen Fragment (scFv), das spezifisch an B-Lymphozyten-Antigen CD19 (CD19) bindet, und unter der Transkriptionskontrolle des Promotors des humanen Elongationsfaktors 1 alpha (EF1a); und
eine Nucleinsäuresequenz, die für das HP-NAP codiert, mit einem künstlichen Signalpeptid zur Sekretion und unter der Transkriptionskontrolle eines induzierbaren NFAT-IL-2-Promotors, der sechs Wiederholungen von Bindemotiven des Kernfaktors aktivierter T-Zellen (NFAT) umfasst.

6. T-Zelle, umfassend:
i) einen chimären Antigenrezeptor (CAR), einen T-Zell-Rezeptor (TCR), eine Nucleinsäuresequenz, die für den CAR codiert, und/oder eine Nucleinsäuresequenz, die für den TCR codiert; und
ein *Helicobacter-pylori*-Neutrophil-aktivierendes Protein (HP-NAP), ein immunologisch äquivalentes Fragment des HP-NAP, eine Nucleinsäuresequenz, die für das HP-NAP codiert, und/oder eine Nucleinsäuresequenz, die für das immunologisch äquivalente Fragment des HP-NAP codiert, wobei das immunologisch äquivalente Fragment des HP-NAP ein Fragment ist, das aktive Polypeptiddomänen von mindestens 20-40 Aminosäureresten des HP-NAP einschließt, die bevorzugt aus der Gruppe ausgewählt sind, bestehend aus SEQ ID NO: 9 bis 12.

7. T-Zelle nach Anspruch 6, wobei der CAR und/oder der TCR spezifisch an ein Antigen bindet, bevorzugt ein Tumorassoziiertes Antigen (TAA) und stärker bevorzugt ausgewählt aus einer Gruppe, bestehend aus B-Lymphozyten-Antigen CD19 (CD19), CD20, Mucin 1 (MUC1), Mesothelin (MSLN), NY-ESO-1, Alpha-Fetoprotein (AFP), carcinoembryonalem Antigen (CEA), Rezeptor 2 des humanen epidermalen Wachstumsfaktors (HER2), Tumorprotein p53 (p53), Ras-Protein (RAS), Melanom-assoziiertem Antigen (MAGE), bevorzugt CD19.

8. T-Zelle nach Anspruch 6 oder 7, wobei das HP-NAP bei Bindung eines Antigens, bevorzugt eines Tumor-assoziierten Antigens (TAA), an den CAR und/oder den TCR durch die T-Zelle exprimiert und sezerniert wird.

9. T-Zelle nach einem der Ansprüche 6 bis 8, wobei die T-Zelle Folgendes umfasst: ii) die Nucleinsäuresequenz, die für das HP-NAP codiert, und/oder die Nucleinsäuresequenz, die für das immunologisch äquivalente Fragment des HP-NAP codiert, unter der Transkriptionskontrolle eines induzierbaren Promotors, der bevorzugt bei Bindung eines Antigens, bevorzugt eines Tumor-assoziierten Antigens (TAA), an den CAR und/oder den TCR aktiviert wird und bei dem es sich stärker bevorzugt um N Bindemotive des Kernfaktors aktivierter T-Zellen (NFAT) handelt, gefolgt vom minimalen Promotor des humanen Interleukin-2 (IL-2), wobei N 1-10 ist, bevorzugt 4-8, stärker bevorzugt 6.

10. T-Zelle nach einem der Ansprüche 6 bis 9, wobei die T-Zelle Folgendes umfasst: i) die Nucleinsäuresequenz, die für den CAR codiert, und/oder die Nucleinsäuresequenz, die für den TCR codiert, unter der Transkriptionskontrolle eines Promotors, der aus einer Gruppe ausgewählt ist, bestehend aus dem Promotor des humanen Elongationsfaktors 1 alpha (EF1a), dem Promotor des Cytomegalovirus (CMV) und dem CAG-Promotor, bevorzugt des EF1a-Promotors.

11. Pharmazeutische Zusammensetzung, umfassend:
eine T-Zelle nach einem der Ansprüche 6 bis 10; und
eine dendritische Zelle, bevorzugt eine unreife dendritische Zelle.

12. T-Zelle nach einem der Ansprüche 6 bis 10 oder pharmazeutische Zusammensetzung nach Anspruch 11 zur Verwendung als Medikament.

13. T-Zelle nach einem der Ansprüche 6 bis 10 oder pharmazeutische Zusammensetzung nach Anspruch 11 zur Verwendung in der T-Zell-Immuntherapie.

14. T-Zelle nach einem der Ansprüche 6 bis 10 oder pharmazeutische Zusammensetzung nach Anspruch 11 zur Verwendung beim Behandeln, Reduzieren und/oder Vorbeugen von Krebs, bevorzugt eines Krebses mit einem soliden Tumor.

15. Verfahren zum Induzieren der Reifung unreifer dendritischer Zellen, wobei das Verfahren Kontaktieren der unreifen dendritischen Zellen in vitro mit einer T-Zelle nach einem der Ansprüche 6 bis 10 umfasst.

## Revendications

1. Vecteur comprenant :
une séquence d'acide nucléique codant pour un récepteur d'antigène chimérique (CAR) et/ou une séquence d'acide nucléique codant pour un récepteur de lymphocyte T (TCR) ; et
une séquence d'acide nucléique codant pour une protéine d'activation des neutrophiles d'Helicobacter pylori (HP-NAP) et/ou une séquence d'acide nucléique codant pour un fragment immunologiquement équivalent de ladite HP-NAP, dans lequel ledit fragment immunologiquement équivalent de ladite HP-NAP est un fragment comportant des domaines polypeptidiques actifs d'au moins 20 à 40 résidus d'acides aminés de ladite HP-NAP, de préférence choisis dans le groupe consistant en SEQ ID NO : 9 à 12.

2. Vecteur selon la revendication 1, dans lequel ledit vecteur est un vecteur viral, de préférence choisi dans un groupe constitué par un vecteur hybride, un vecteur lentiviral, un vecteur adénoviral, un vecteur viral adéno-associé et un vecteur rétroviral, plus préférentiellement un vecteur lentiviral.

3. Vecteur selon la revendication 1 ou 2, dans lequel ladite séquence d'acide nucléique codant pour ladite HP-NAP et/ou ladite séquence d'acide nucléique codant pour ledit fragment immunologiquement équivalent de ladite HP-NAP est sous contrôle transcriptionnel d'un promoteur inductible, de préférence activé sur la liaison d'un antigène, de préférence un antigène associé à une tumeur (TAA), audit CAR et/ou audit TCR, et de manière davantage préférée, étant des motifs de liaison du facteur nucléaire N des lymphocytes T activés (NFAT) suivis du promoteur minimal de l'interleukine 2 humaine (IL-2), N est 1-10, de préférence 4-8, plus préférablement 6.

4. Vecteur selon l'une quelconque des revendications 1 à 3, dans lequel ladite séquence d'acide nucléique codant pour ledit CAR et/ou ladite séquence d'acide nucléique codant pour ledit TCR est sous contrôle transcriptionnel d'un promoteur choisi dans un groupe constitué par le promoteur facteur d'élongation alpha 1 humain (EF1a), le promoteur du cytomégalovirus (CMV) et le promoteur CAG, de préférence le promoteur EF1a.

5. Vecteur selon l'une quelconque des revendications 1 à 4, comprenant :
une séquence d'acide nucléique codant pour ledit CAR ayant un fragment variable à chaîne unique dérivé d'un anticorps extracellulaire (scFv) qui se lie spécifiquement à l'antigène CD19 des lymphocytes B (CD19) et sous contrôle transcriptionnel du promoteur facteur d'élongation 1 alpha humain (EF1a) ; et
une séquence d'acide nucléique codant pour ladite HP-NAP avec un peptide signal artificiel pour la sécrétion et sous contrôle transcriptionnel d'un promoteur NFAT-IL-2 inductible comprenant six répétitions de motifs de liaison au facteur nucléaire des lymphocytes T activés (NFAT).

6. Lymphocyte T comprenant :
i) un récepteur d'antigène chimérique (CAR), un récepteur de lymphocyte T (TCR), une séquence d'acide nucléique codant pour ledit CAR et/ou une séquence d'acide nucléique codant pour ledit TCR ; et
une protéine d'activation des neutrophiles d'Helicobacter pylori (HP-NAP), un fragment immunologiquement équivalent de ladite HP-NAP, une séquence d'acide nucléique codant pour ladite HP-NAP et/ou une séquence d'acide nucléique codant pour ledit fragment immunologiquement équivalent de ladite HP-NAP, dans lequel ledit fragment immunologiquement équivalent de ladite HP-NAP est un fragment comportant des domaines polypeptidiques actifs d'au moins 20 à 40 résidus d'acides aminés de ladite HP-NAP, de préférence choisis dans le groupe consistant en SEQ ID NO : 9 à 12.

7. Lymphocyte T selon la revendication 6, dans lequel ledit CAR et/ou ledit TCR se lie spécifiquement à un antigène, de préférence un antigène associé à une tumeur (TAA), et de manière davantage préférée choisi dans un groupe constitué par l'antigène lymphocytaire B CD19 (CD19), CD20, mucine 1 (MUC1), mésothéline (MSLN), NY-ESO-1, alpha-foetoprotéine (AFP), antigène carcinoembryonnaire (CEA), récepteur 2 du facteur de croissance épidermique humain (HER2), protéine tumorale p53 (p53), protéine Ras (RAS), antigène associé au mélanome (MAGE), de préférence CD19.

8. Lymphocyte T selon la revendication 6 ou 7, dans lequel ladite HP-NAP est exprimée et sécrétée par ledit lymphocyte T lors de la liaison d'un antigène, de préférence un antigène associé à une tumeur (TAA), audit CAR et/ou audit TCR.

9. Lymphocyte T selon l'une quelconque des revendications 6 à 8, dans lequel ledit lymphocyte T comprend ii) ladite séquence d'acide nucléique codant pour ladite HP-NAP et/ou ladite séquence d'acide nucléique codant pour ledit fragment immunologiquement équivalent de ladite HP-NAP sous contrôle transcriptionnel d'un promoteur inductible, de préférence activé lors de la liaison d'un antigène, de préférence un antigène associé à une tumeur (TAA), audit CAR et/ou audit TCR, et de manière davantage préférée, étant des motifs de liaison du facteur nucléaire N des lymphocytes T activés (NFAT) motifs suivis du promoteur minimal de l'interleukine humaine 2 (IL-2), N est 1-10, de préférence 4-8, plus préférablement 6.

10. Lymphocyte T selon l'une quelconque des revendications 6 à 9, dans lequel ledit lymphocyte T comprend i) ladite séquence d'acide nucléique codant pour ledit CAR et/ou ladite séquence d'acide nucléique codant pour ledit TCR sous contrôle transcriptionnel d'un promoteur choisi dans un groupe constitué par le promoteur facteur d'élongation 1 alpha humain (EF1a), le promoteur du cytomégalovirus (CMV) et le promoteur CAG, de préférence le promoteur EF1a.

11. Composition pharmaceutique comprenant :
un lymphocyte T selon l'une quelconque des revendications 6 à 10 ; et
une cellule dendritique, de préférence une cellule dendritique immature.

12. Lymphocyte T selon l'une quelconque des revendications 6 à 10 ou composition pharmaceutique selon la revendication 11 pour une utilisation en tant que médicament.

13. Lymphocyte T selon l'une quelconque des revendications 6 à 10 ou composition pharmaceutique selon la revendication 11 destinée à être utilisée dans l'immunothérapie des lymphocytes T.

14. Lymphocyte T selon l'une quelconque des revendications 6 à 10 ou composition pharmaceutique selon la revendication 11 pour une utilisation dans le traitement, la réduction et/ou la prévention du cancer, de préférence un cancer à tumeur solide.

15. Procédé d'induction de la maturation de cellules dendritiques immatures, ledit procédé comprenant la mise en contact in vitro desdites cellules dendritiques immatures avec un lymphocyte T selon l'une quelconque des revendications 6 à 10.
